(19) Europäisches Patentamt
European Patent Office
Office européen des brevets

(11) **EP 4 751 701 A1**

(12) **EUROPEAN PATENT APPLICATION**
published in accordance with Art. 153(4) EPC

(43) Date of publication:
03.06.2026 Bulletin 2026/23

(21) Application number: 24845500.8

(22) Date of filing: 17.07.2024

(51) International Patent Classification (IPC):
*A61K 8/04* (2006.01)          *A61K 8/19* (2006.01)
*A61K 8/31* (2006.01)          *A61K 8/34* (2006.01)
*A61K 8/37* (2006.01)          *A61K 8/58* (2006.01)
*A61K 8/891* (2006.01)          *A61K 8/894* (2006.01)
*A61Q 17/04* (2006.01)          *A61Q 19/00* (2006.01)

(52) Cooperative Patent Classification (CPC):
A61K 8/04; A61K 8/19; A61K 8/31; A61K 8/34;
A61K 8/37; A61K 8/58; A61K 8/891; A61K 8/894;
A61Q 17/04; A61Q 19/00

(86) International application number:
PCT/JP2024/025657

(87) International publication number:
WO 2025/023121 (30.01.2025 Gazette 2025/05)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: 26.07.2023 JP 2023121608

(71) Applicant: **SHIN-ETSU CHEMICAL CO., LTD.
Tokyo 100-0005 (JP)**

(72) Inventor: **KONISHI Masayuki
Tokyo 100-0005 (JP)**

(74) Representative: **Mewburn Ellis LLP
Aurora Building
Counterslip
Bristol BS1 6BX (GB)**

(54) **DISPERSION, PRODUCTION METHOD FOR SAME, AND COSMETIC**

(57) A dispersion according to the present invention contains components (A)-(D) and has not only favorable dispersion properties immediately after production but also favorable viscosity stability (dispersion stability).
(A) A nonvolatile oil that has a kinematic viscosity of 1-100 mm²/s at 25°C: 15-65 mass%.

(B) A powder: 30-70 mass%.
(C) A dispersant that is soluble in component (A): 1-15 mass%.
(D) A volatile component selected from among lower alcohols and volatile oils that have a boiling point of no more than 250°C: 100 ppm-10 mass%.

EP 4 751 701 A1

**Description**

TECHNICAL FIELD

The present invention relates to a dispersion, a method for producing the same, and a cosmetic containing the dispersion. In the present invention, compositions for use in cosmetics are referred to as cosmetics.

BACKGROUND ART

As known in the art, dispersants are conventionally used to enhance the wettability and the dispersibility of powders in cosmetics and thereby offer enhancements in the feeling on use and in storage stability (Patent Document 1). It is known that the dispersing performance can be enhanced through the study of the structural design of dispersants.
On the other hand, dispersants are highly susceptible to the influence of dispersion media. This fact makes the selection of a dispersion medium and a dispersant important. For example, it is known that a highly polar oil or the like has poor compatibility with a silicone dispersant, but a composition with good dispersion properties can be obtained by selecting a silicone dispersant having an alkyl branch (Patent Document 2). However, no research has been conducted on adsorption properties of dispersants.

PRIOR ART DOCUMENTS

PATENT DOCUMENTS

Patent Document 1: WO 2016/178380
Patent Document 2: WO 2017/199732

SUMMARY OF INVENTION

TECHNICAL PROBLEM

The present invention has been made in view of the circumstances discussed above. It is therefore an object of the present invention to provide a dispersion that has good dispersion properties immediately after production and also exhibits good viscosity stability (dispersion stability), and a cosmetic that contains the dispersion without any deterioration in favorable properties of the cosmetic caused by the addition of the dispersion, and has excellent over-time stability.

SOLUTION TO PROBLEM

As a result of extensive studies directed to achieving the above object, the present inventor has found that a dispersion including (A) a nonvolatile oil having a kinematic viscosity at 25°C of 1 to 100 mm$^2$/s, (B) a powder, (C) a dispersant soluble in the ingredient (A), and (D) a volatile ingredient selected from lower alcohols and volatile oils having a boiling point of 250°C or below, has good adsorption of the dispersant to the powder and can solve the problems described above. The present invention has been completed based on the finding.
Thus, the present invention provides the following inventive aspects.

1. A dispersion including ingredients (A), (B), (C), and (D) below:

   (A) a nonvolatile oil having a kinematic viscosity at 25°C of 1 to 100 mm$^2$/s: 15 to 65% by mass,
   (B) a powder: 30 to 70% by mass,
   (C) a dispersant soluble in the ingredient (A): 1 to 15% by mass, and
   (D) a volatile ingredient selected from lower alcohols and volatile oils having a boiling point of 250°C or below: 100 ppm to 10% by mass.

2. The dispersion according to 1, wherein the ingredient (B) is dispersed with the ingredients (C) and (D), and the ingredient (B) thus dispersed is further dispersed in the ingredient (A).
3. The dispersion according to 1 or 2, which has a viscosity change rate after 2 weeks at 25°C of less than 250% relative to a viscosity immediately after preparation.
4. The dispersion according to any one of 1 to 3, wherein the ingredient (A) is one or more selected from ester oils, phenyl silicones, and alkyl silicones.

5. The dispersion according to any one of 1 to 4, wherein the ingredient (B) has an average primary particle size of 8 to 200 nm.

6. The dispersion according to any one of 1 to 5, wherein the ingredient (B) is hydrophobized microparticulate titanium oxide.

7. The dispersion according to any one of 1 to 6, wherein the ingredient (C) is a silicone surfactant.

8. The dispersion according to any one of 1 to 7, wherein the ingredient (D) includes one or more selected from isododecane, undecane, tridecane, cyclopentasiloxane, disiloxane, trisiloxane, dimethicone, and methyltrimethicone.

9. A method for producing the dispersion described in any one of 1 to 8, the method including:

a step of dispersing the ingredient (B) with the ingredients (C) and (D) to prepare a preliminary dispersion; and
a step of mixing the preliminary dispersion with the ingredient (A).

10. A cosmetic including the dispersion described in any one of 1 to 8.

11. The cosmetic according to 10, wherein the cosmetic is an oil-in-water cosmetic.

12. The cosmetic according to 10 or 11, further including (E) an organic ultraviolet absorber.

ADVANTAGEOUS EFFECTS OF INVENTION

The dispersion according to the present invention has excellent powder dispersibility and viscosity stability (dispersion stability). The dispersion can be added to a cosmetic without impairing favorable properties of the cosmetic, and the resultant cosmetic has excellent over-time stability.

DESCRIPTION OF EMBODIMENTS

The present invention will be described in detail below without limiting the scope of the present invention to the following description. In the present invention, the name of an ingredient is written as the name cited in *Keshōhin Hyōji Meishō* [Japanese Cosmetic Labeling Names] or in the International Nomenclature of Cosmetic Ingredients (INCI). When the names from *Keshōhin Hyōji Meishō* and the INCI are the same, the name from *Keshōhin Hyōji Meishō* or the INCI name is sometimes omitted.

[Ingredients (A)]

The ingredient (A) in the present invention is a nonvolatile oil having a kinematic viscosity at 25°C of 1 to 100 $mm^2$/s. Such ingredients may be used singly, or two or more may be used in appropriate combination. In the present invention, the kinematic viscosity is a value measured at 25°C with a Cannon-Fenske viscometer in accordance with the method described in JIS Z 8803: 2011. The kinematic viscosity is 1 to 100 $mm^2$/s (cSt; sometimes written as cs), and is preferably 1 to 50 $mm^2$/s, and more preferably 1 to 30 $mm^2$/s.

In the present invention, a nonvolatile oil is an oil having a boiling point higher than 250°C. Specific examples include silicone oils, hydrocarbon oils, ester oils, higher fatty acids, natural oils, and fluorochemical oils. Among these, one or more selected from ester oils, phenyl silicones, and alkyl silicones are preferable because excellent compatibility is obtained when an organic ultraviolet absorber is added to a cosmetic.

• Silicone oils

Examples of the silicone oils include phenyl silicones, such as phenyl trimethicone (labeling name, INCI: Phenyl Trimethicone), diphenyl dimethicone (labeling name, INCI: Diphenyl Dimethicone), and diphenylsiloxy phenyl trimethicone (labeling name, INCI: Diphenylsiloxy Phenyl Trimethicone), alkyl silicones, such as caprylyl methicone (labeling name, INCI: Caprylyl Methicone), linear or branched dimethicones, such as low-viscosity dimethicone (labeling name, INCI: Dimethicone), hydrogen dimethicone (labeling name, INCI: Hydrogen Dimethicone), and the like that have a kinematic viscosity of 1 to 100 $mm^2$/s, amodimethicone (labeling name, INCI: Amodimethicone), and aminopropyl dimethicone (labeling name, INCI: Aminopropyl Dimethicone). Among these, in particular, exemplary commercially available silicone oils include KF-4418, KF-96A-6cs, KF-56A, and KF-50-100cs manufactured by Shin-Etsu Chemical Co., Ltd. Examples further include amino acid-modified silicones and fluorine-modified silicones.

• Ester oils

The ester oils are liquid oils that are condensation products of a C1-C20 fatty acid and a C1-C20 alcohol and may have an animal-derived or vegetable-derived composition. Examples include monoesters and polyesters, such as diesters and triesters. Specific examples include diisobutyl adipate (labeling name, INCI: Diisobutyl Adipate), dihexyldecyl adipate (labeling name), diheptylundecyl adipate (labeling name, INCI: Diheptylundecyl Adipate), n-alkyl glycol monoisostearates, such as isostearyl isostearate (labeling name, INCI: Isostearyl Isostearate), isocetyl isostearate (labeling name, INCI: Isocetyl Isostearate), glycol diethylhexanoate (labeling name, INCI: Glycol Diethylhexanoate), cetyl ethylhexanoate (labeling name, INCI: Cetyl Ethylhexanoate), triethylhexanoin (labeling name, INCI: Triethylhexanoin), trimethylolpropane triethylhexanoate (labeling name, INCI: Trimethylolpropane Triethylhexanoate), oleyl oleate (labeling name, INCI: Oleyl Oleate), octyldodecyl oleate (labeling name, INCI: Octyldodecyl Oleate), decyl oleate (labeling name, INCI: Decyl Oleate), neopentyl glycol diethylhexanoate (labeling name, INCI: Neopentyl Glycol Diethylhexanoate), neopentyl glycol dicaprate (labeling name, INCI: Neopentyl Glycol Dicaprate), triethyl citrate (labeling name, INCI: Triethyl Citrate), diethylhexyl succinate (labeling name, INCI: Diethylhexyl Succinate), amyl acetate (labeling name, INCI: Amyl Acetate), ethyl acetate (labeling name, INCI: Ethyl Acetate), butyl acetate (labeling name, INCI: Butyl Acetate), isocetyl stearate (labeling name, INCI: Isocetyl Stearate), butyl stearate (labeling name, INCI: Butyl Stearate), diisopropyl sebacate (labeling name, INCI: Diisopropyl Sebacate), diethylhexyl sebacate (labeling name, INCI: Diethylhexyl Sebacate), isononyl isononanoate (labeling name, INCI: Isononyl Isononanoate), isotridecyl isononanoate (labeling name, INCI: Isotridecyl Isononanoate), cocoalkyl (caprylate/caprate) (labeling name, INCI: Coco-Caprylate/Caprate), glyceryl tri(caprylate/caprate) (labeling name, INCI: Caprylic/Capric Triglyceride), palmitic acid esters, such as isopropyl palmitate (labeling name, INCI: Isopropyl Palmitate), ethylhexyl palmitate (labeling name, INCI: Ethylhexyl Isopalmitate), and hexyldecyl palmitate (labeling name, INCI: Isocetyl Palmitate, Hexyldecyl Palmitate), myristic acid esters, such as isopropyl myristate (labeling name, INCI: Isopropyl Myristate) and octyldodecyl myristate (labeling name, INCI: Octyldodecyl Myristate), ethylhexyl laurate (labeling name, INCI: Ethylhexyl Laurate), hexyl laurate (labeling name, INCI: Hexyl Laurate), and isopropyl lauroyl sarcosinate (labeling name, INCI: Isopropyl Lauroyl Sarcosinate).

• Hydrocarbon oils

Examples of the hydrocarbon oils include linear or branched nonvolatile hydrocarbon oils. Specific examples include hydrogenated polyisobutene (labeling name, INCI: Hydrogenated Polyisobutene), Squalane (INCI), and Mineral Oil (INCI).

In particular, one or more selected from ester oils, phenyl silicones, and alkyl silicones are preferable.

The amount in which the ingredient (A) is added is 15 to 65% by mass of the dispersion, and is preferably 20 to 55% by mass, and more preferably 40 to 50% by mass. If the amount is less than 15% by mass, the dispersion will be highly viscous and difficult to handle. If the amount is more than 65% by mass, the dispersion may be unstable or may fail to achieve desired effects, such as UV-shielding effect.

[Ingredients (B)]

The ingredient (B) in the present invention is a powder. The ingredient (B) may be any material without limitation that can be normally added to cosmetics. Examples include microparticulate metal oxide powders, coloring pigments, inorganic powders, metal powders, organic powders, and inorganic-organic composite powders. The powders may be used singly, or two or more may be used in appropriate combination. Each of these powders will be described in detail below. A microparticulate powder having an average primary particle size of 8 to 200 nm is preferable, and the average primary particle size is more preferably 120 nm or less. If the particle size is larger than the above range, the UV-protecting function is lowered and the powder applied leaves a white cast on the skin. The average primary particle size is the average of 200 particles and can be determined from transmission electron microphotographs. The powder may take any shape, with examples including spherical, roughly spherical, bar, spindle, petal, strip, and irregular shapes.

• Microparticulate metal oxide powders

The microparticulate metal oxide used in the present invention is one, or two or more selected from titanium oxide, for example, microparticulate titanium oxide (labeling name, INCI: Titanium Dioxide), iron-containing titanium oxide, zinc oxide (labeling name, INCI: Zinc Oxide), cerium oxide (labeling name, INCI: Cerium Oxide), and composites thereof. A composite powder of the above metal oxide with other powder may also be used. The microparticulate metal oxide powder is added for the purpose of imparting a UV-protecting function to the dispersion of the present invention or a cosmetic containing the dispersion. The average primary particle size is preferably 8 to 200 nm, and more preferably 120 nm or less. If the particle size is larger than the above range, the UV-protecting function is lowered and the powder applied leaves a white cast on the skin.

The microparticulate metal oxide may be untreated or may have undergone well-known surface treatment generally used for cosmetics. For example, inorganic treatments include coating with silica (labeling name, INCI: Silica), coating with alumina (labeling name, INCI: Alumina), and coating with Al hydroxide (labeling name, INCI: Aluminum Hydroxide); and organic treatments include silanes or silylating agents, such as triethoxycaprylylsilane (labeling name, INCI: Triethoxycaprylylsilane), silicone oils, such as dimethicone (labeling name, INCI: Dimethicone), hydrogen dimethicone (labeling name, INCI: Hydrogen Dimethicone), and triethoxysilylethyl polydimethylsiloxyethyl hexyl dimethicone (labeling name, INCI: Triethoxysilylethyl Polydimethylsiloxyethyl Hexyl Dimethicone), waxes, paraffins, organic fluorine compounds, such as perfluoroalkyl phosphate salts, surfactants, amino acids, such as N-acylglutamic acid, and metal soaps, such as Al stearate (labeling name, INCI: Aluminum Stearate) and Mg myristate (labeling name, INCI: Magnesium Myristate). In particular, triethoxysilylethyl polydimethylsiloxyethyl hexyl dimethicone (labeling name, INCI: Triethoxysilylethyl Polydimethylsiloxyethyl Hexyl Dimethicone) offers high dispersibility in both silicones and oils, and is therefore applied to sunscreens and foundations. Treatment with triethoxycaprylylsilane (labeling name, INCI: Triethoxycaprylylsilane) and metal soaps is preferable in terms of the compatibility with the ingredients (A), (C), and (D). These surface treatments may be applied singly, or two or more may be combined depending on the purpose.

Microparticulate metal oxides that are surface-treated as described above are available in the market. For example, microparticulate titanium oxide is commercially available under the trade names of STR-100C-LP, STR-100A-LP, STR-100W, STR-100W-LP, STR-100C-LF, and STR-40-LP (manufactured by SAKAI CHEMICAL INDUSTRY CO., LTD.), MT-N1, MT-01, MT-05, MT-100Z, MT-100TV, MT-100AQ, MT-100WP, MTY-100SAS, MT-150EX, MT-500B, MT-505SAS, MT-700B, MT-700Z, MT-014Z, and SMT-500SAS (manufactured by TAYCA Co., Ltd.), and ST-455, ST-455WS, ST-457ECS, and ST-495M (manufactured by Titan Kogyo, Ltd.). Microparticulate zinc oxide is commercially available under the trade names of FINEX-50S-LP2, FINEX-30S-LP2, FINEX-50W, FINEX-30W, FINEX-50W-LP2, FINEX-52W-LP2, FINEX-30W-LP2, FINEX-33W-LP2, FINEX-50-LPT, FINEX-25-LPT, FINEX-50S-LPT, and FINEX-30S-LPT (manufactured by SAKAI CHEMICAL INDUSTRY CO., LTD.), and MZ-150, MZ-200, MZ-300, MZ-306X, MZX-303S, MZX-303M, MZX-304OTS, MZ-500HP, MZ-505T, MZX-505HPS, MZY-505M, MZ-506X, MZY-203S, MZX-203OTS, MZY-210M3S, TMZ-HA1, and MZX-5080TS (manufactured by TAYCA Co., Ltd.).

• Coloring pigments

The coloring pigment is not particularly limited and may be any pigment commonly used in cosmetics for the purpose of coloring. Examples include red iron oxide (labeling name, INCI: Iron Oxides), yellow iron oxide (labeling name, INCI: Iron Oxides), white titanium oxide (labeling name, INCI: Titanium Dioxide), black iron oxide (labeling name, INCI: Iron Oxides), ultramarine (labeling name, INCI: Ultramarines), Prussian blue (labeling name, INCI: Ferric Ferrocyanide, Ferric Ammonium Ferrocyanide), manganese violet (labeling name, INCI: Manganese Violet), cobalt titanate (labeling name, INCI: Cobalt Titanium Oxide), chromium hydroxide (labeling name, INCI: Chromium Hydroxide Green), chromium oxide (labeling name, INCI: Chromium Oxide Greens), Al/cobalt oxide (labeling name, INCI: Cobalt Aluminum Oxide), fired titanium/titanium oxide (labeling name, INCI: Titanium/Titanium Dioxide), Li/cobalt titanate (labeling name, INCI: Lithium Cobalt Titanate), sintered iron oxide/titanium oxide (labeling name, Iron Oxide/Titanium Dioxido Sinter), composites doped with a different metal, such as iron oxide-doped titanium oxide (labeling name, INCI: Iron Oxides, Titanium Dioxide), titanium nitride (labeling name, INCI: Titanium Nitride), ferrous hydroxide (labeling name, INCI: Iron Hydroxide), inorganic brown pigments, such as $\gamma$-iron oxide, inorganic yellow pigments, such as loess, and colored pigments, such as lake form of tar dyes and lake form of natural dyes. Any of the foregoing may be used. The coloring pigments may be surface-treated. Hydrophobized coloring pigments are preferable in terms of dispersibility.

The shape or geometry of the coloring pigment is not particularly limited as long as a color can be imparted to a cosmetic. In view of hiding power, a pigment having a particle size, specifically, a volume average particle size in the range of 150 to 600 nm is preferable. The volume average particle size may be measured by TEM or the like. If the volume average particle size is less than 150 nm, the hiding power is so low that the cosmetic may have low coloring efficiency. If the volume average particle size is larger than 600 nm, the feeling on use may be deteriorated.

The pigment used in the present invention may be treated on part or the entirety of its surface with an inorganic compound, such as alumina (labeling name, INCI: Alumina), Al hydroxide (labeling name, INCI: Aluminum Hydroxide), silica (labeling name, INCI: Silica), or hydrous silica (labeling name, INCI: Hydrated Silica).

The hydrophobizing treatment for the hydrophobized coloring pigment is surface treatment of the coloring pigment with a hydrophobizing agent. The hydrophobizing agent for the surface of the coloring pigment in the present invention is not particularly limited as long as the agent can impart hydrophobicity. Exemplary treating agents include silicone treating agents, waxes, paraffins, organic fluorine compounds, such as perfluoroalkyl phosphate salts, surfactants, amino acids, such as N-acylglutamic acid, and metal soaps, such as Al stearate (labeling name, INCI: Aluminum Stearate) and Mg myristate (labeling name, INCI: Magnesium Myristate).

In particular, silicone treating agents are preferably used. Examples include silanes or silylating agents, such as triethoxycaprylylsilane (labeling name, INCI: Triethoxycaprylylsilane) and trimethoxysilyl dimethicone (labeling name, INCI: Trimethoxysilyl Dimethicone); silicone oils, such as dimethicone (labeling name, INCI: Dimethicone), hydrogen dimethicone (labeling name, INCI: Hydrogen Dimethicone), and triethoxysilylethyl polydimethylsiloxyethyl hexyl dimethicone (labeling name, INCI: Triethoxysilylethyl Polydimethylsiloxyethyl Hexyl Dimethicone); and silicone compounds, such as (acrylates/tridecyl acrylate/triethoxysilylpropyl methacrylate/dimethicone methacrylate) copolymer (labeling name, INCI: Acrylates/Tridecyl Acrylate/Triethoxysilylpropyl Methacrylate/Dimethicone Methacrylate Copolymer), and (acrylates/dimethicone) copolymer (labeling name, INCI: Acrylates/Dimethicone Copolymer). Suitable silicone treating agents are silicone powder treating agents described in JP 3912961. In particular, for example, triethoxysilylethyl polydimethylsiloxyethyl hexyl dimethicone (labeling name, INCI: Triethoxysilylethyl Polydimethylsiloxyethyl Hexyl Dimethicone) that is a dimethylpolysiloxane having a triethoxysilyl group, a polydimethylsiloxyethyl group, and a hexyl group on side chains is effectively used because this compound exerts high affinity even when the dispersion medium in which the highly hydrophobized coloring pigment is dispersed is a mixture of, for example, a silicone and a hydrocarbon. The surface hydrophobizing agents may be used singly, or two or more may be used in combination.

In the present invention, the coloring pigment may be surface-treated with the hydrophobizing agent by any known production method without limitation. The surface treatment methods are largely classified into dry process and wet process. In a dry process, for example, the treatment may be performed by mixing/contacting the coloring pigment to be used in the present invention with the hydrophobizing agent using an appropriate stirring machine, crusher, mixer, disperser or the like, such as a Henschel mixer, a ball mill, a jet mill, a kneader, a planetary mixer, a sand mill, an attritor, a ribbon blender, a dispersing mixer, or a homomixer. In this process, the treatment may be performed while applying energy, such as heat, mechanochemical mechanical force, or overheated steam. The treatment may also be performed in such a manner that the coloring pigment and the hydrophobizing agent are sufficiently mixed/contacted and thereafter energy, such as heat, mechanochemical mechanical force, or overheated steam, is separately applied. In order to enhance the efficiency in dispersing the hydrophobizing agent in the process of mixing/contacting the hydrophobizing agent with the coloring pigment, for example, the hydrophobizing agent may be dissolved or dispersed beforehand in an appropriate amount of water, solvent, or supercritical fluid, and the resultant solution or dispersion may be sprayed to the coloring pigment. In a wet process, the treatment may be performed in such a manner that the coloring pigment and the hydrophobizing agent are dispersed in water, solvent, or supercritical fluid, thereby being mixed/contacted with each other, thereafter the solvent is evaporated, and further energy, such as heat, mechanochemical mechanical force, or overheated steam, is separately applied.

Specific examples of the hydrophobized coloring pigments include KTP-09 series, especially KTP-09W, KTP-09R, KTP-09Y, and KTP-09B (manufactured by Shin-Etsu Chemical Co., Ltd.).

• Inorganic powders

Examples of the inorganic powders include microparticles of zirconium oxide (labeling name, INCI: Zirconium Dioxide), zinc oxide (labeling name, INCI: Zinc Oxide), cerium oxide (labeling name, INCI: Cerium Oxide), Mg oxide (labeling name, INCI: Magnesium Oxide), Ba sulfate (labeling name, INCI: Barium Sulfate), calcium sulfate (labeling name, INCI: Calcium Sulfate), Mg sulfate (labeling name, INCI: Magnesium Sulfate), Ca carbonate (labeling name, INCI: Calcium Carbonate), Mg carbonate (labeling name, INCI: Magnesium Carbonate), talc (labeling name, INCI: Talc), cleaved talc (labeling name, INCI: Talc), mica (labeling name, INCI: Mica), kaolin (labeling name, INCI: Kaolin), sericite (labeling name, INCI: Mica), synthetic fluorphlogopite (labeling name, INCI: Synthetic Fluorphlogopite), black mica (labeling name, INCI: Biotite), K silicate (labeling name, INCI: Potassium Silicate), silica (labeling name, INCI: Silica), fumed silica, Al silicate (labeling name, INCI: Aluminum Silicate), Mg silicate (labeling name, INCI: Magnesium Silicate), Al/Mg silicate (labeling name, INCI: Magnesium Aluminum Silicate), Ca silicate (labeling name, INCI: Calcium Silicate), Al/Ca/Na silicate (labeling name, INCI: Aluminum Calcium Sodium Silicate), Li/Mg/Na silicate (labeling name, INCI: Lithium Magnesium Sodium Silicate), Na/Mg silicate (labeling name, INCI: Sodium Magnesium Silicate), Ca/Al borosilicate (labeling name, INCI: Calcium Aluminum Borosilicate), Ca/Na borosilicate (labeling name, INCI: Calcium Sodium Borosilicate), hydroxyapatite (labeling name, INCI: Hydroxyapatite), bentonite (labeling name, INCI: Bentonite), montmorillonite (labeling name, INCI: Montmorillonite), hectorite (labeling name, INCI: Hectorite), zeolite (labeling name, INCI: Zeolite), alumina (labeling name, INCI: Alumina), Al hydroxide (labeling name, INCI: Aluminum Hydroxide), boron nitride (labeling name, INCI: Boron Nitride), and glass (labeling name, INCI: Glass). Furthermore, examples of the inorganic coloring pearly pigments include pearly agents, such as titanium oxide-coated mica; and pearly pigments, such as bismuth oxychloride (labeling name, INCI: Bismuth Oxychloride), bismuth oxychloride (labeling name, INCI: Bismuth Oxychloride) coated with titanium oxide (labeling name, INCI: Titanium Dioxide), talc (labeling name, INCI: Talc) coated with titanium oxide (labeling name, INCI: Titanium Dioxide), fish scale flakes, and coloring mica coated with titanium oxide (labeling name, INCI: Titanium Dioxide). These may be untreated or may have undergone well-known surface treatment generally used for cosmetics.

• Metal powders

Examples of the metal powders include metal microparticles of aluminum (labeling name, INCI: Aluminum Powder), copper (labeling name, INCI: Copper Powder), and silver (labeling name, INCI: Silver Powder).

• Organic powders

Examples of the organic powders include powders of silicones, polyamides, polyacrylic acids-acrylates, polyesters, polyethylene, polypropylene, polystyrene, styrene-acrylic acid copolymers, divinylbenzene-styrene copolymers, polyurethanes, vinyl resins, urea resins, melamine resins, benzoguanamine, polymethyl benzoguanamine, tetrafluoroethylene, polymethyl methacrylate (such as, for example, polymethyl methacrylate), celluloses, silk, nylons, phenolic resins, epoxy resins, and polycarbonate. In particular, examples of the silicones include silicone resin particles (such as, specifically, polymethylsilsesquioxane (labeling name, INCI: Polymethylsilsesquioxane) and silicone resin-coated silicone rubber powders (specifically, (vinyl dimethicone/methicone silsesquioxane) crosspolymer (labeling name, INCI: Vinyl Dimethicone/Methicone Silsesquioxane Crosspolymer), (diphenyl dimethicone/vinyl diphenyl dimethicone/-silsesquioxane) crosspolymer (labeling name, INCI: Diphenyl Dimethicone/Vinyl Diphenyl Dimethicone/Silsesquioxane Crosspolymer), polysilicone-1 crosspolymer (labeling name, INCI: Polysilicone-1 Crosspolymer), and polysilicone-22 (labeling name, INCI: Polysilicone-22). Examples further include powders of metal soaps, specifically, zinc stearate (labeling name, INCI: Zinc Stearate), Al stearate (labeling name, INCI: Aluminum Stearate), Ca stearate (labeling name, INCI: Calcium Stearate), Mg stearate (labeling name, INCI: Magnesium Stearate), zinc myristate (labeling name, INCI: Zinc Myristate), Mg myristate (labeling name, INCI: Magnesium Myristate), zinc/Na cetylphosphate (labeling name, INCI: Sodium Zinc Cetyl Phosphate), and K cetylphosphate (labeling name, INCI: Potassium Cetyl Phosphate). Examples further include organic colorants, specifically, tar dyes, such as Red #3, Red #104(1) (labeling name, INCI: Red 28, Red 28 Lake), Red #106, Red #201 (labeling name, INCI: Red 6), Red #202 (labeling name, INCI: Red 7), Red #204, Red #205, Red #220 (labeling name, INCI: Red 34), Red #226 (labeling name, INCI: Red 30), Red #227 (labeling name, INCI: Red 33, Red 33 Lake), Red #228 (labeling name, INCI: Red 36), Red #230(1) (labeling name, INCI: Red 22, Red 22 Lake), Red #230(2), Red #401, Red #505, Yellow #4 (labeling name, INCI: Yellow 5), Yellow #5 (labeling name, INCI: Yellow 6, Yellow 6 Lake), Yellow #202(1) (labeling name, INCI: Yellow 8), Yellow #203 (labeling name, INCI: Yellow 10, Yellow 10 Lake), Yellow #204 (labeling name, INCI: Yellow 11), Yellow #401 (labeling name, INCI:), Blue #1 (labeling name, INCI: Blue 1, Blue 1 Lake), Blue #2, Blue #201, Blue #205 (labeling name, INCI: Blue 4), Blue #404, Green #3 (labeling name, INCI: Green 3, Green 3 Lake), Green #201 (labeling name, INCI: Green 5), Green #202 (labeling name, INCI: Green 6), Green #204 (labeling name, INCI: Green 8), Green #205, Orange #201 (labeling name, INCI: Orange 5), Orange #203 (labeling name, INCI: Pigment Orange 5), Orange #204, Orange #205 (labeling name, INCI: Orange 4, Orange 4 Lake), Orange #206 (labeling name, INCI: Orange 10), and Orange #207 (labeling name, INCI: Orange 11); and natural dyes, such as cochineal (labeling name, INCI: Cochineal), laccaic acid (labeling name, INCI: Laccaic Acid), safflower red (labeling name, INCI: Carthamus Tinctorius (Safflower) Flower Extract), red root extract (labeling name, INCI: Lithospermum Officinale Root Extract), gardenia yellow, and gardenia blue (labeling name, INCI: Hydrolyzed Gardenia Florida Extract).

• Inorganic/organic composite powders

Examples of the inorganic/organic composite powders include composite powders obtained by coating the surface of an inorganic powder with an organic powder by any well-known method.

Among the ingredients (B), microparticulate metal oxide powders and hydrophobized coloring pigments that require dispersibility are preferable. Hydrophobized microparticulate titanium oxide, such as metal soap-treated microparticulate titanium oxide, is more preferable.

The amount in which the ingredient (B) is added is 30 to 70% by mass of the dispersion, and is preferably 40 to 70% by mass, and more preferably 40 to 60% by mass. If the amount is less than 30% by mass, the desired effects, such as sufficient UV-shielding effects and coloring effects, cannot be obtained. If the amount is more than 70% by mass, the spreadability at the time of use may be deteriorated, or a cosmetic film may be whitish or powdered.

[Ingredients (C)]

The ingredient (C) in the present invention is a dispersant that is soluble in the ingredient (A). The ingredient (C) may be any dispersant without limitation that can be normally added to cosmetics and is soluble in the ingredient (A). The ingredients (C) may be used singly, or two or more may be used in appropriate combination.

Regarding the phrase "soluble in the ingredient (A)" in the present invention, the term "soluble" means that when the ingredient (C) is mixed with the ingredient (A) at a concentration of 20% by mass and is then allowed to stand at 25°C for 1 hour, the mixture is transparent to semi-transparent without any boundaries. If the mixture is white turbid or has separated into two layers, the ingredient is "insoluble". The transparency is the total light transmittance measured with respect to a 1 cm thick cell filled with the sample in accordance with the method described in JIS K7361-1: 1997. The mixture is judged to be transparent to semi-transparent when the total light transmittance is 50% or more.

Silicone surfactants are preferable from the point of view of the dispersibility in the ingredient (A), such as an ester oil or a silicone oil. For example, one or more silicone compounds selected from the group consisting of modified silicones and reactive silicones may be used. In particular, polyether-modified silicones, polyglycerin-modified silicones, acrylic silicones, and amino-modified silicones are preferable from the point of view of dispersing effects. Among these, acrylic silicones and polyglycerin-modified silicones are more preferable due to their higher dispersing effects. In the chemical structure of the polyglycerin-modified silicones, the silicone main chain may be modified with a polyglycerin block in the main chain or may be modified with a polyglycerin branch. In order to maintain uniform dispersibility of the ingredient (B) in the dispersion, a branch-modified silicone is more preferable. The silicone main chain may have a branched chain, such as a silicone chain. Specific examples include Polyglyceryl-3 Polydimethylsiloxyethyl Dimethicone (INCI), Lauryl Polyglyceryl-3 Polydimethylsiloxyethyl Dimethicone (INCI), and Bis-Butyl Dimethicone Polyglyceryl-3 (INCI). Examples of commercially available polyglycerin-modified silicones include KF-6105, KF-6104, KF-6106, and KF-6115 manufactured by Shin-Etsu Chemical Co., Ltd. Examples of commercially available acrylic silicones include KP-578 manufactured by Shin-Etsu Chemical Co., Ltd. Examples of commercially available polyether-modified silicones include KF-6017, KF-6028, KF-6038, and KF-6048 manufactured by Shin-Etsu Chemical Co., Ltd.

The amount in which the ingredient (C) is added is 1 to 15% by mass of the dispersion, and is preferably 3 to 15% by mass, more preferably 3 to 12% by mass, and still more preferably 5 to 10% by mass. If the amount is less than 1% by mass, dispersion stability may be deteriorated. If the amount is more than 15% by mass, molecules of the ingredient (C) may be oriented on the ingredient (B) that already has molecules of the ingredient (C) oriented thereon, or molecules of the ingredient (C) that have been oriented on the ingredient (B) may be easily detached by other molecules of the ingredient (C). Possible consequences are an increased viscosity of the dispersion and adverse effects on dispersion properties and dispersion stability.

[Ingredients (D)]

The ingredient (D) in the present invention is a volatile ingredient selected from lower alcohols and volatile oils having a boiling point of 250°C or below. Such ingredients may be used singly, or two or more may be used in combination.

The lower alcohols include C1-C4 alcohols, such as, for example, ethanol, propanol, isopropanol, butyl alcohol, and isobutyl alcohol.

In the present invention, the volatile oils are oils that have a boiling point of 250°C or below. Specific examples include linear (poly)siloxanes, such as disiloxane, trisiloxane (KF-96L-1cs, manufactured by Shin-Etsu Chemical Co., Ltd.), and dimethylpolysiloxane (KF-96L-1.5cs, KF-96L-2cs, manufactured by Shin-Etsu Chemical Co., Ltd.), cyclic polysiloxanes, such as cyclotetrasiloxane (D4), cyclopentasiloxane (D5) (KF-995, manufactured by Shin-Etsu Chemical Co., Ltd.), and cyclohexasiloxane (D6), branched polysiloxanes, such as methyltrimethicone (TMF-1.5, manufactured by Shin-Etsu Chemical Co., Ltd.), and hydrocarbon solvents, such as undecane, isododecane, C13-15 alkane, and C9-12 alkane, each having a boiling point of 250°C or below.

From the point of view of dispersibility, the ingredient (D) is preferably apolar or low-polar. In particular, from the points of view of over-time stability and feel when used in cosmetics, a volatile ingredient(s) selected from isododecane, undecane, tridecane, cyclopentasiloxane, disiloxane, trisiloxane, dimethicone (KF-96L-1.5cs, KF-96L-2cs, manufactured by Shin-Etsu Chemical Co., Ltd.), and methyltrimethicone is preferable. That is, it is preferable that the ingredient(s) (D) be or include any of these. The above ingredients may be used singly, or two or more may be used in appropriate combination.

The amount in which the ingredient (D) is added is 100 ppm (by mass) to 10% by mass of the dispersion, and is preferably 100 ppm to 8.5% by mass, more preferably 100 ppm to 5% by mass, and still more preferably 100 ppm to 1.5% by mass. This amount ensures that the dispersion that is obtained will be further enhanced in powder dispersibility and viscosity stability (dispersion stability).

[Dispersions]

The present invention pertains to a dispersion containing the above-described ingredients (A), (B), (C), and (D), the powder (B) being dispersed. The total amount of the ingredients (A) to (D) in the dispersion is preferably 98 to 100% by mass, preferably 99 to 100% by mass, or preferably 100% by mass with substantially no optional ingredients. Some exemplary optional ingredients are antioxidants and water. From the points of view of over-time stability and properties when added to cosmetics, the dispersion is preferably such that the ingredient (B) is dispersed with the ingredients (C) and (D), and the ingredient (B) thus dispersed is further dispersed in the ingredient (A).

The dispersion may be a liquid or a paste. The initial viscosity at 25°C immediately after production is not particularly limited. When the production is made with a medium agitating mill, such as a bead mill, the initial viscosity is preferably less than 1,000 mPa·s in view of the ease of filtering the beads and the ease of blending into a cosmetic. From the point of view of feeling on use, the initial viscosity is more preferably less than 750 mPa·s, and still more preferably less than 500 mPa·s. The lower limit is not particularly limited, but the concentration variation in the dispersion tends to be small when the initial viscosity is 100 mPa·s or more. The dispersion is analyzed using a Brookfield viscometer with, for example, Spindle LV-2 at 30 rotations, and the viscosity is measured after 60 seconds. When the dispersion is a paste, the hardness is not particularly limited. When the production is made with a three-roll mill, the hardness is preferably 100 or less to facilitate the processing. The hardness is more preferably less than 80, and still more preferably less than 50 to facilitate blending in cosmetics. The lower limit is not particularly limited, but the concentration variation in the dispersion tends to be small when the hardness is 5 or more. The hardness is a value measured with a rheometer, for example, Rheometer RT-2002D·D (manufactured by RHEOTECH, probe: 10 mmφ, penetration depth: 10 mm, sample stage rising speed: 5 cm/min, temperature: 25°C, range: 200) (the same applies hereinafter).

In order to maintain the stability of a cosmetic containing the dispersion, it is preferable that the dispersion have a viscosity change rate after 2 weeks at 25°C of less than 250% relative to the viscosity immediately after preparation of the dispersion. This viscosity change rate is determined by the method described in Examples. The viscosity change rate at 25°C after 2 weeks at 25°C is more preferably less than 200%, still more preferably 160% or less, and particularly preferably 140% or less. The lower limit of the viscosity change rate is not particularly limited, but may be 40% or more, 80% or more, or 90% or more. The viscosity of the dispersion at 25°C after 2 weeks at 25°C is preferably 40 mPa·s or more and less than 2,500 mPa·s, and more preferably 80 to 2,000 mPa·s.

<Methods for producing the dispersion>

The dispersion of the present invention may be produced by a known method. From the point of view of the adsorption of the ingredient (C) to the ingredient (B), in particular, the production method preferably includes a step of dispersing the ingredient (B) with the ingredients (C) and (D) to prepare a preliminary dispersion; and a step of mixing the preliminary dispersion obtained above with the ingredient (A). The step of mixing the preliminary dispersion with the ingredient (A) may be, for example, the following step (I), (II), or (III).

(I) A production process that includes a step of adding the ingredient (A) or a mixed solvent including the ingredients (A) and (D) to the preliminary dispersion and further dispersing the mixture. In the process, the amounts of the ingredients are adjusted so as to satisfy the proportions described hereinabove.

(II) A production process that includes a step of mixing the preliminary dispersion with the ingredient (A), and subjecting the mixture to a reduced pressure and an elevated temperature to volatilize the ingredient (D). In the process, the amounts of the ingredients are adjusted so as to satisfy the proportions described hereinabove.

(III) A production process that includes adding a mixed solvent including the ingredients (A) and (C), or the ingredients (A), (C), and (D) to the preliminary dispersion and further dispersing the mixture. In the process, the amounts of the ingredients are adjusted so as to satisfy the proportions described hereinabove.

While the ingredient (D) may be volatilized before the ingredient (A) is mixed, the above processes are preferable in order to obtain a dispersion with higher dispersion properties.

For example, the dispersing process may involve any stirrer, grinder, mixer, medium agitating machine, planetary centrifugal stirrer, or disperser, such as Henschel mixer, ball mill, kneader, planetary mixer, ribbon blender, disperser mixer, homomixer, jet mill, roll mill, bead mill, or high-pressure disperser. From the point of view of mixing efficiency, in particular, it is preferable that the ingredients be dispersed using a bead mill or a high-pressure disperser.

[Cosmetics]

The present invention provides a cosmetic including the dispersion described above. The amount of the dispersion in the cosmetic is appropriately selected from 0.5 to 60% by mass, and is preferably 5 to 31% by mass.

The present invention is applicable to a variety of cosmetics, and is preferably applied to cosmetics externally applied to the skin, such as skin care cosmetics, makeup cosmetics, antiperspirant cosmetics, and UV-protecting cosmetics, to cosmetics externally applied to the hair, such as hair care cosmetics, and to nail cosmetics. Exemplary skin care cosmetics include skin lotions, milky lotions, creams, cleansing agents, packs, oil liquids, massage creams, cosmetic liquids, cosmetic oils, detergents, deodorants, hand creams, lip creams, and anti-wrinkle agents. Exemplary makeup cosmetics include makeup bases, foundations, concealers, cosmetic powders, cheek colors, eye colors, eye shadows, mascaras, eye liners, eyebrows, and lip cosmetics. Exemplary antiperspirant cosmetics include antiperspirants of roll-on type, cream type, solution type, and stick type. Exemplary UV-protecting cosmetics include sunscreen oils, sunscreen milky lotions, sunscreen creams, and preparations obtained by imparting sunscreen effects to the above makeup cosmetics. Exemplary hair care cosmetics include shampoos, conditioners, treatments, setting agents, hair dyes, and hair fragrances.

The type of the cosmetic of the present invention may be any of, for example, powder, oily liquid, non-aqueous, emulsion, such as water-in-oil or oil-in-water emulsion, or multi-emulsion, such as W/O/W or O/W/O emulsion. The form of the cosmetic of the present invention may be selected from various forms including liquid, milky lotion, cream, solid, paste, gel, powder, pressed, multilayer, mousse, spray, stick, and pencil. In particular, the present invention is more effective in an oil-in-water cosmetic because it is generally difficult to disperse a powder in such a cosmetic.

[Ingredients (E)]

The cosmetic of the present invention may contain an ingredient (E): an organic ultraviolet absorber. Some examples of the ingredients (E) are illustrated below, but any such ingredients generally added to cosmetics may be used without limitation. The ingredients may be used singly, or two or more may be used in appropriate combination.

Specific examples include oxybenzone-1 (labeling name, INCI: Benzophenone-1), oxybenzone-2 (labeling name, INCI: Benzophenone-2), oxybenzone-3 (labeling name, INCI: Benzophenone-3), oxybenzone-4 (labeling name, INCI: Benzophenone-4), oxybenzone-5 (labeling name, INCI: Benzophenone-5), oxybenzone-6 (labeling name, INCI: Benzophenone-6), oxybenzone-9 (labeling name, INCI: Benzophenone-9), Homosalate (INCI), Octocrylene (INCI), t-butyl methoxydibenzoylmethane (labeling name, INCI: Butyl Methoxydibenzoylmethane), ethylhexyl salicylate (labeling name, INCI: Ethylhexyl Salicylate), diethylaminohydroxybenzoyl hexyl benzoate (labeling name, INCI: Diethylamino Hydroxybenzoyl Hexyl Benzoate), Polysilicone-15 (INCI), dimethoxybenzylidene dioxoimidazolidine octyl propionate (labeling name, INCI: Ethylhexyl Dimethoxybenzylidene Dioxoimidazolidine Propionate), terephthalylidene dicamphor sulfonic acid (labeling name, INCI: Terephthalylidene Dicamphor Sulfonic Acid), Ethylhexyl Triazone (INCI), methyl-bis(trimethylsiloxy)silyl isopentyl trimethoxycinnamate (labeling name, INCI: Isopentyl Trimethoxycinnamate Trisiloxane), Drometrizole Trisiloxane (INCI), dimethyl PABA ethylhexyl (labeling name, INCI: Ethylhexyl Dimethyl PABA), isopropyl para-methoxycinnamate (labeling name, INCI: Isopropyl Methoxycinnamate), ethylhexyl methoxycinnamate (labeling name, INCI: Ethylhexyl Methoxycinnamate), Bis-Ethylhexyloxyphenol Methoxyphenyltriazine (INCI), phenylbenzimidazole sulfonic acid (labeling name, INCI: Phenylbenzimidazole Sulfonic Acid), Methylene Bis-Benzotriazolyl Tetramethylbutylphenol (INCI), glyceryl ethylhexanoate dimethoxycinnamate (labeling name, INCI: Glyceryl Ethylhexanoate Dimethoxycinnamate), Glyceryl PABA (INCI), methyl diisopropylcinnamate (labeling name, INCI: Diisopropyl Methyl Cinnamate), Cinoxate (INCI), and ethylhexyl dimethoxybenzylidene dioxoimidazolidine propionate (labeling name, INCI: Ethylhexyl Dimethoxybenzylidene Dioxoimidazolidine Propionate). Furthermore, a UVA absorber (such as, for example, Diethylamino Hydroxybenzoyl Hexyl Benzoate) and a UVB absorber (such as, for example, Ethylhexyl Methoxycinnamate) may be used in combination, or absorbers belonging to the same type may be combined appropriately.

When the organic ultraviolet absorber is added, the amount thereof is not particularly limited but is preferably 0.5 to 30% by mass, more preferably 3 to 25% by mass, and still more preferably 5 to 20% by mass of the whole cosmetic. If the amount is more than 30% by mass, the cosmetic may feel heavy or may be highly irritating. Because the dispersion of the present invention has excellent compatibility with organic ultraviolet absorbers, the present invention produces greater effects when the amount is 0.5% by mass or more. The cosmetic that contains the dispersion of the present invention can attain high stability even when, in particular, the concentration of an organic ultraviolet absorber in the oil phase is high, for example, 8% by mass or more, 10% by mass or more, or 15% by mass or more of the whole cosmetic.

The cosmetic of the present invention may contain various ingredients that are usually added to cosmetics without impairing the advantageous effects of the present invention. For example, such additional ingredients are (1) oils, (2) aqueous ingredients, (3) surfactants, (4) powders, (5) compositions consisting of a crosslinked organopolysiloxane and an oil that is liquid at room temperature, (6) film-forming agents, (7) UV-absorbing/scattering agents, and (8) other additives. These may be each used singly, or two or more may be used in appropriate combination. The ingredients contained in the dispersion described hereinabove may be added. However, the marked effects aimed at in the present invention cannot be obtained even if the ingredients (A) to (D) of the present invention are added unless the dispersion of the present invention is added. The dispersion of the present invention allows even a powder that is difficult to disperse in a cosmetic, such as a coloring pigment or a microparticulate powder, to be dispersed easily. Thus, the amount of the ingredient (C) added in the cosmetic, excluding the ingredient (C) in the dispersion, may be 0 to 8% by mass, 0.1 to 5% by mass, or 0.1 to 3% by mass.

(1) Oils

The cosmetic of the present invention may contain an oil that does not belong to the ingredients (A) or the ingredients (D). The oils may be volatile or nonvolatile and may be solid, semisolid, or liquid at room temperature (25°C).

EP 4 751 701 A1

Examples include silicone oils, silicone waxes, natural animal and plant oils and fats, semi-synthetic oils and fats, hydrocarbon oils, higher alcohols, fatty acids, ester oils, and fluorochemical oils.

• Silicone oils

Examples of the silicone oils include Dimethicone (INCI); Trisiloxane (INCI); alkyl-modified silicones, such as Methyl Trimethicone (INCI), Ethyl Trisiloxane (INCI), Ethyl Methicone (INCI), and Hexyl Dimethicone (INCI); long-chain alkyl-modified silicones, such as Caprylyl Methicone (INCI); medium- to high-viscosity, linear or branched organopolysiloxanes, such as Phenyl Trimethicone (INCI), Diphenyl Dimethicone (INCI), Diphenylsiloxyphenyl Trimethicone (INCI), Tetraphenyl Dimethyldisiloxane (INCI), and Methylhydrogenpolysiloxane; cyclic organopolysiloxanes, such as Cyclotetrasiloxane (INCI), Cyclopentasiloxane (INCI), and Cyclohexasiloxane (INCI); amino-modified organopolysiloxanes, such as Amodimethicone (INCI) and Aminopropyl Dimethicone (INCI); pyrrolidone-modified organopolysiloxanes, such as PCA Dimethicone (INCI); pyrrolidone carboxylic acid-modified organopolysiloxanes; silicone rubbers, such as gum-like dimethylpolysiloxanes having a high degree of polymerization, gum-like amino-modified organopolysiloxanes, and gum-like dimethylsiloxane-methylphenylsiloxane copolymers; as well as low-viscosity organopolysiloxane solutions of silicone gums and rubbers; amino acid-modified silicones; fluorine-modified silicones; silicone resins; and silicone resin solutions.
Examples of commercially available silicone oils include KF-96L-1cs, KF-96L-1.5cs, KF-96L-2cs, KF-96A-6cs, TMF-1.5, KF-4422, KF-4418, KF-56A, KF-995, KF-54, and KF-54HV manufactured by Shin-Etsu Chemical Co., Ltd.

• Solid oil ingredients

When a solid cosmetic is desired in the present invention, it is preferable to add an oil ingredient that is solid at 25°C. The oil ingredient that is solid at 25°C is preferably one having a melting point of 40°C or above, more preferably 60 to 110°C. Examples include waxes, hydrocarbons, esters, higher alcohols, and higher fatty acids. The solid oil ingredients are not particularly limited and may be any materials usually added to cosmetics. Specific examples include plant waxes, such as carnauba wax (INCI: Copernicia Cerifera (Carnauba) Wax), sugar cane wax, candelilla wax (INCI: Euphorbia Cerifera (Candelilla) Wax), purified candelilla wax, rice wax, Japan wax, jojoba wax, kapok wax, rice bran wax, myrica cerifera fruit wax, shea butter, cacao butter, Japan wax (INCI: Rhus Succedanea Fruit Wax), montan wax (INCI: Montan Wax), and hydrogenated castor oil isostearate; animal waxes, such as beeswax, beef tallow, beef bone fat, lard (INCI: Lard), horse fat (INCI: Horse Fat), mutton tallow, lanolin (INCI: Lanolin), Ericerus pela wax, shellac wax, and sperm wax; semi-synthetic waxes, such as lanolin esters, lanolin fatty acid esters, and beeswax acid esters; hydrogenated oils, such as hydrogenated castor oil and hydrogenated coconut oil; hydrocarbon waxes, such as solid paraffin, polyethylene, ceresin, ozokerite, and microcrystalline wax; wax esters, such as synthetic beeswax; dioctyldodecyl lauroylglutamate, fatty acids, such as stearic acid and behenic acid; silicone waxes, such as acrylate silicone resins in the form of acrylate silicone graft or block copolymers (such as acrylate silicone graft copolymers: KP-561P and KP-562P manufactured by Shin-Etsu Chemical Co., Ltd.); and derivatives thereof. Any one, or two or more selected from the foregoing are preferable.

• Natural animal and plant oils and semi-synthetic oils

Examples of the natural animal and plant oils and the semi-synthetic oils include natural plant oils, such as avocado oil (labeling name, INCI: Persea Gratissima (Avocado) Oil), linseed oil (labeling name, INCI: Linum Usitatissimum (Linseed) Seed Oil), almond oil (labeling name, INCI: Prunus Amygdalus Dulcis (Sweet Almond) Oil), perilla oil (labeling name), olive oil (labeling name, INCI: Olea Europaea (Olive) Fruit Oil), Torreya californica oil (labeling name, INCI: Torreya Californica (California Nutmeg) Oil), citronella grass oil (labeling name, INCI: Cymbopogon Nardus (Citronella) Oil), Torreya Nucifera seed oil (labeling name, INCI: Torreya Nucifera Seed Oil), apricot kernel oil (labeling name, INCI: Kyounin Yu), wheat germ oil (labeling name, INCI: Triticum Vulgare (Wheat) Germ Oil), sesame oil (labeling name, INCI: Sesamum Indicum (Sesame) Seed Oil), rice germ oil (labeling name, INCI: Oryza Sativa (Rice) Germ Oil), rice bran oil (labeling name, INCI: Oryza Sativa (Rice) Bran Oil), sasanqua oil (labeling name, INCI: Camellia Kissi Seed Oil), safflower oil (labeling name, INCI: Carthamus Tinctorius (Safflower) Seed Oil), soybean oil (labeling name, INCI: Glycine Soja (Soybean) Oil), tea seed oil (labeling name, INCI: Camellia Sinensis Seed Oil), camellia oil (labeling name, INCI: Camellia Japonica Seed Oil), evening primrose oil (labeling name, INCI: Oenothera Biennis (Evening Primrose) Oil), rapeseed oil (labeling name), germ oils, for example, corn germ oil (labeling name, INCI: Zea Mays (Corn) Germ Oil), persic oil (labeling name), palm oil (labeling name, INCI: Elaeis Guineensis (Palm) Oil), palm kernel oil (labeling name, INCI: Elaeis Guineensis (Palm) Kernel Oil), castor oil (labeling name, INCI: Ricinus Communis (Castor) Seed Oil), sunflower seed oil (labeling name, INCI: Helianthus Annuus (Sunflower) Seed Oil), grape seed oil (labeling name, INCI: Vitis Vinifera (Grape) Seed Oil), jojoba seed oil (labeling name, INCI: Simmondsia Chinensis (Jojoba) Seed Oil), macadamia seed oil (labeling name, INCI: Macadamia Ternifolia Seed Oil), meadowfoam oil (labeling name, INCI: Limnanthes Alba (Meadowfoam) Seed Oil), cotton seed oil (labeling name, INCI: Gossypium Herbaceum (Cotton) Seed Oil), coconut oil (labeling name, INCI: Cocos Nucifera (Coconut) Oil), and peanut oil (labeling name, INCI: Arachis Hypogaea (Peanut) Oil); natural animal oils, such as shark liver oil (labeling name, INCI: Shark Liver Oil), cod liver oil (labeling name, INCI: Cod Liver Oil), fish liver oil (labeling name, INCI: Fish Liver Oil), turtle oil (labeling name, INCI: Turtle Oil), mink oil (labeling name, INCI: Mink Oil), and egg yolk oil (labeling name, INCI: Egg Oil); and semi-synthetic oils and fats, such as hydrogenated coconut oil (labeling name, INCI: Hydrogenated Coconut Oil) and liquid lanolin (labeling name, INCI: Lanolin Oil).

• Hydrocarbon oils

Examples of the hydrocarbon oils include linear or branched, nonvolatile hydrocarbon oils. Specific examples include alkanes, such as Olefin Oligomer (INCI), isoparaffins, for example, (C13, 14) Isoparaffin (INCI), Isododecane (INCI), Undecane (INCI), Dodecane (INCI), Isohexadecane (INCI), hydrogenated polyisobutene (labeling name, INCI: Hydrogenated Polyisobutene), Squalane (INCI), Mineral Oil (INCI), Coconut Alkane (INCI), and (C13-15) Alkane (INCI).

• Higher alcohols

Examples of the higher alcohols include linear saturated alcohols having 6 or more carbon atoms, such as Lauryl Alcohol (INCI), Hexyldecanol (INCI), Oleyl Alcohol (INCI), Isostearyl Alcohol (INCI), Octyldodecanol (INCI), Decyltetradecanol (INCI), Myristyl Alcohol (INCI), Cetyl Alcohol (INCI), Stearyl Alcohol (INCI), and Behenyl Alcohol (INCI), as well as Batyl Alcohol (INCI). Examples further include sterols, such as Cholesterol (INCI), sitosterol (labeling name, INCI: Beta-Sitosterol)), Phytosterols (INCI), and Lanosterol (INCI).

• Ester oils

Examples of the ester oils include diisobutyl adipate (labeling name, INCI: Diisobutyl Adipate), dihexyldecyl adipate (labeling name), diheptylundecyl adipate (labeling name, INCI: Diheptylundecyl Adipate), alkylglycol monoisostearates, such as isostearyl isostearate (labeling name, INCI: Isostearyl Isostearate), isocetyl isostearate (labeling name, INCI: Isocetyl Isostearate), trimethylolpropane triisostearate (labeling name, INCI: Trimethylolpropane Triisostearate), glycol diethylhexanoate (labeling name, INCI: Glycol Diethylhexanoate), cetyl ethylhexanoate (labeling name, INCI: Cetyl Ethylhexanoate), trimethylolpropane triethylhexanoate (labeling name, INCI: Trimethylolpropane Triethylhexanoate), pentaerythrityl tetraethylhexanoate (labeling name, INCI: Pentaerythrityl Tetraethylhexanoate), octyldodecyl esters, such as octyldodecyl stearoyloxystearate (labeling name, INCI: Octyldodecyl Stearoyl Stearate), oleyl oleate (labeling name, INCI: Oleyl Oleate), octyldodecyl oleate (labeling name, INCI: Octyldodecyl Oleate), decyl oleate (labeling name, INCI: Decyl Oleate), neopentyl glycol diethylhexanoate (labeling name, INCI: Neopentyl Glycol Diethylhexanoate), neopentyl glycol dicaprate (labeling name, INCI: Neopentyl Glycol Dicaprate), diisostearyl malate (labeling name, INCI: Diisostearyl Malate), triethyl citrate (labeling name, INCI: Triethyl Citrate), diethylhexyl succinate (labeling name, INCI: Diethylhexyl Succinate), amyl acetate (labeling name, INCI: Amyl Acetate), ethyl acetate (labeling name, INCI: Ethyl Acetate), butyl acetate (labeling name, INCI: Butyl Acetate), isocetyl stearate (labeling name, INCI: Isocetyl Stearate), butyl stearate (labeling name, INCI: Butyl Stearate), diisopropyl sebacate (labeling name, INCI: Diisopropyl Sebacate), diethylhexyl sebacate (labeling name, INCI: Diethylhexyl Sebacate), cetyl lactate (labeling name, INCI: Cetyl Lactate), myristyl lactate (labeling name, INCI: Myristyl Lactate), isononyl isononanoate (labeling name, INCI: Isononyl Isononanoate), isotridecyl isononanoate (labeling name, INCI: Isotridecyl Isononanoate), palmitates, such as isopropyl palmitate (labeling name, INCI: Isopropyl Palmitate), ethylhexyl palmitate (labeling name, INCI: Ethylhexyl Isopalmitate), and hexyldecyl palmitate (labeling name, INCI: Isocetyl Palmitate, Hexyldecyl Palmitate), cholesteryl hydroxystearate (labeling name, INCI: Cholesteryl Hydroxystearate), myristates, such as isopropyl myristate (labeling name, INCI: Isopropyl Myristate), octyldodecyl myristate (labeling name, INCI: Octyldodecyl Myristate), and myristyl myristate (labeling name, INCI: Myristyl Myristate), ethylhexyl laurate (labeling name, INCI: Ethylhexyl Laurate), hexyl laurate (labeling name, INCI: Hexyl Laurate), dioctyldodecyl lauroyl glutamate (labeling name, INCI: Dioctyldodecyl Lauroyl Glutamate), isopropyl lauroyl sarcosinate (labeling name, INCI: Isopropyl Lauroyl Sarcosinate), coco-alkyl caprylate/caprate (labeling name, INCI: Coco-Caprylate/Caprate), diisostearyl malate (labeling name, INCI: Diisostearyl Malate), phytosteryl isostearate (labeling name, INCI: Phytosteryl Isostearate), di(phytosteryl/octyldodecyl) lauroyl glutamate (labeling name, INCI: Phytosteryl/Octyldodecyl Lauroyl Glutamate), and diesters of dilinoleic acid, phytosterols, and higher alcohols.
Among the ester oils, exemplary glyceride oils include Triethylhexanoin (INCI), glyceryl tri(caprylate/caprate) (labeling name, INCI: Caprylic/Capric Triglyceride), Cocoglyceryl (INCI), triglyceryl caprylate/caprate/succinate (labeling name, INCI: Caprylic/Capric/Succinic Triglyceride), and caprylic/capric glycerides (labeling name, INCI: Caprylic/Capric Glycerides).

• Fluorochemical oils

Examples of the fluorochemical oils include Perfluorodecalin (INCI), Perfluorononyl Dimethicone (INCI), and Perfluoromethylcyclopentane (INCI).

(2) Aqueous ingredients

The aqueous ingredients are not particularly limited as long as they are aqueous ingredients that are usually added to cosmetics. Specific examples include water, lower alcohols preferably having 2 to 5 carbon atoms, such as ethanol (labeling name, INCI: Alcohol) and isopropanol (labeling name, INCI: Isopropyl Alcohol), and sugar alcohols, such as Sorbitol (INCI), Maltose (INCI), and Xylitol (INCI). Examples further include polyhydric alcohols, such as BG (labeling name, INCI: Butylene Glycol), PG (labeling name, INCI: Propylene Glycol), DPG (labeling name, INCI: Dipropylene Glycol), Pentylene Glycol (INCI), 1,10-Decanediol (INCI), Octanediol (INCI), 1,2-Hexanediol (INCI), Erythritol (INCI), Glycerin (INCI), Diglycerin (INCI), and polyethylene glycol; and moisturizers, such as Glucose (INCI), Glyceryl Glucoside (INCI), Betaine (INCI), Na chondroitin sulfate (labeling name, INCI: Sodium Chondroitin Sulfate), PCA-Na (labeling name, INCI: Sodium PCA), Methyl Gluceth-10 (INCI), Methyl Gluceth-20 (INCI), hyaluronic acid, egg yolk lecithin, soybean lecithin, phosphatidylcholine, phosphatidylethanolamine, phosphatidylserine, phosphatidylglycerol, phosphatidylinositol, and sphingophospholipid.

(3) Surfactants

The surfactants may be nonionic, anionic, cationic, or ampholytic. The surfactants are not particularly limited and any surfactants usually used in cosmetics may be used. Among such surfactants, one, or two or more selected from non-crosslinked silicone surfactants and crosslinked silicone surfactants are preferable because stable cosmetics can be obtained. For any types of surfactants, the amount is preferably 0.1 to 20% by mass of the whole cosmetic. An amount of 0.1% by mass or more ensures that the dispersing and emulsifying functions are fully achieved whereas an amount of 20% by mass or less advantageously eliminates the risk that the cosmetic gives a sticky feeling on use. The HLB of the surfactants is not limited but is preferably 2 to 14.5 in order to maintain the water resistance of the cosmetic.

The non-crosslinked silicone surfactants are linear or branched silicones in which some methyl groups on the backbone are substituted with hydrophilic groups, such as polyethylene glycol or polyglycerin. Specifically, preferred non-crosslinked silicone surfactants are linear or branched polyoxyethylene-modified organopolysiloxanes, linear or branched polyoxyethylene polyoxypropylene-modified organopolysiloxanes, linear or branched polyoxyethylene/alkyl-co-modified organopolysiloxanes, linear or branched polyoxyethylene polyoxypropylene/alkyl-co-modified organopolysiloxanes, linear or branched polyglycerin-modified organopolysiloxanes, linear or branched polyglycerin/alkyl-co-modified organopolysiloxanes, and linear or branched pyrrolidone-modified organopolysiloxanes. Examples include PEG-11 Methyl Ether Dimethicone (INCI), PEG/PPG-20/22 Butyl Ether Dimethicone (INCI), PEG-3 Dimethicone (INCI), PEG-10 Dimethicone (INCI), PEG-9 Polydimethylsiloxyethyl Dimethicone (INCI), Lauryl PEG-9 Polydimethylsiloxyethyl Dimethicone (INCI), Cetyl PEG/PPG-10/1 Dimethicone (INCI), Polyglyceryl-3 Disiloxane Dimethicone (INCI), Polyglyceryl-3 Polydimethylsiloxyethyl Dimethicone (INCI), Lauryl Polyglyceryl-3 Polydimethylsiloxyethyl Dimethicone (INCI), and Bis-Butyl Dimethicone Polyglyceryl-3 (INCI).

Examples of commercially available products include KF-6011, KF-6011P, KF-6012, KF-6015, KF-6017, KF-6043, KF-6028, KF-6038, KF-6048, KF-6100, KF-6104, KF-6106, KF-6105, KF-6115, and KF-6180 manufactured by Shin-Etsu Chemical Co., Ltd.

Examples of the crosslinked silicone surfactants include crosslinked polyether-modified silicones, such as (Dimethicone/(PEG-10/15)) Crosspolymer (INCI), (PEG-15/Lauryl Dimethicone) Crosspolymer (INCI), (PEG-10/Lauryl Dimethicone) Crosspolymer (INCI), and (PEG-15/Lauryl Polydimethylsiloxyethyl Dimethicone) Crosspolymer (INCI); and crosslinked polyglycerin-modified silicones, such as (Dimethicone/Polyglycerin-3) Crosspolymer (INCI), (Lauryl Dimethicone/Polyglycerin-3) Crosspolymer (INCI), and (Polyglycerin-3/Lauryl Polydimethylsiloxyethyl Dimethicone) Crosspolymer (INCI).

When the crosslinked silicone surfactant is used as a composition consisting of the crosslinked silicone surfactant and an oil that is liquid at room temperature, it is preferable that the crosslinked silicone surfactant be swollen by containing its own weight or more of the liquid oil.

The liquid oil may be any of the silicone oils, the hydrocarbon oils, the ester oils, the natural animal and plant oils, the semi-synthetic oils, and the fluorochemical oils that are described hereinabove as the optional ingredients, namely, the oils (1), and are liquid, with examples including Cyclopentasiloxane (INCI), Dimethicone (INCI), Mineral Oil (INCI), Isododecane (INCI), Isohexadecane (INCI), Triethylhexanoin (INCI), isotridecyl isononanoate (labeling name, INCI: Isotridecyl Isononanoate), and Squalane (INCI).

Commercially available crosslinked silicone surfactants swollen by containing a liquid oil include, for example, KSG-210, KSG-240, KSG-270, KSG-310, KSG-320, KSG-330, KSG-340, KSG-320Z, KSG-350Z, KSG-710, KSG-790, KSG-810, KSG-820, KSG-830, KSG-840, KSG-820Z, and KSG-850Z manufactured by Shin-Etsu Chemical Co., Ltd.

(4) Powders

The powder is added as the dispersion described hereinabove and may further be added separately. Furthermore, the powder may be further added to the cosmetic as an additional dispersion. Specific examples of dispersions in which UV-absorbing/scattering particles are dispersed in an oil include SPD series (product name), in particular, SPD-T5, T5L, Z5, Z5L, T6, Z6, T7, and Z7L manufactured by Shin-Etsu Chemical Co., Ltd.

The amount of the powder that is added separately other than as the dispersion is preferably 0.5 to 90% by mass, more preferably 1 to 30% by mass, and still more preferably 5 to 20% by mass of the cosmetic.

(5) Compositions consisting of a crosslinked organopolysiloxane and an oil that is liquid at room temperature

In the composition consisting of a crosslinked organopolysiloxane and an oil that is liquid at room temperature, it is preferable that the crosslinked organopolysiloxane be swollen by containing its own weight or more of the liquid oil. The liquid oil may be any of the silicone oils, the hydrocarbon oils, the ester oils, the natural animal and plant oils, the semi-synthetic oils, and the fluorochemical oils that are described hereinabove as the optional ingredients, namely, the oils (1), and are liquid. Examples include Cyclopentasiloxane (INCI), Dimethicone (INCI), Mineral Oil (INCI), Isododecane (INCI), Isohexadecane (INCI), Triethylhexanoin (INCI), isotridecyl isononanoate (labeling name, INCI: Isotridecyl Isononanoate), and Squalane (INCI).

Unlike the crosslinked silicone surfactants described above as the ingredients (3), the ingredients (5) are compounds that do not have a polyether or polyglycerin structure in the molecular structure. Specific examples include Dimethicone/Vinyl Dimethicone Crosspolymer (INCI), Dimethicone/Phenyl Vinyl Dimethicone Crosspolymer (INCI), Vinyl Dimethicone/Lauryl Dimethicone Crosspolymer (INCI), and Lauryl Polydimethylsiloxyethyl Dimethicone/Bis-Vinyl Dimethicone Crosspolymer (INCI).

Examples of commercially available compositions consisting of a crosslinked organopolysiloxane and an oil that is liquid at room temperature include KSG-15, KSG-1510, KSG-16, KSG-1610, KSG-19, KSG-016F, KSG-18A, KSG-41A, KSG-42A, KSG-43, KSG-44, KSG-45, KSG-042Z, KSG-045Z, and KSG-048Z manufactured by Shin-Etsu Chemical Co., Ltd.

(6) Film-forming agents

The film-forming agent is added mainly for the purpose of further extending the durability of the effects of the cosmetic. The film-forming agents are not particularly limited. Silicone compositions are preferable from the point of view of imparting water repellency. Specifically, for example, use may be made of trimethylsiloxysilicic acid, acrylic-silicone film formers, silicone-modified norbornene, silicone-modified pullulan, and silicone-modified polyvinyl alcohol.

Examples of the film-forming agents in the form of silicone compositions include trimethylsiloxysilicic acid (labeling name, INCI: Trimethylsiloxysilicate), Acrylates/Dimethicone Copolymer (INCI), Norbornene/Tris(Trimethylsiloxy)Silylnorbornene Copolymer (INCI), and pullulan tri(trimethylsiloxy)silylpropyl carbamide (labeling name, INCI: Trimethylsiloxysilylcarbamoyl Pullulan).

The film-forming agent may be previously dissolved into an oil that is liquid at room temperature before being added to the cosmetic. The liquid oil may be any of the silicone oils, the hydrocarbon oils, the ester oils, the natural animal and plant oils, the semi-synthetic oils, and the fluorochemical oils that are described hereinabove as the optional ingredients, namely, the oils (1), and are liquid.

Specific examples of commercially available silicone film-forming agents include KF-7312J, KP-545, KP-549, KP-543, NBN-30-ID, TSPL-30-ID, and TSPL-30-D5 manufactured by Shin-Etsu Chemical Co., Ltd.

(7) UV-absorbing/scattering agents

Examples of the UV-absorbing/scattering agents include particles that absorb and scatter ultraviolet rays, such as titanium oxide microparticles, iron-containing titanium oxide microparticles, zinc oxide microparticles, cerium oxide microparticles, and composites thereof. These UV-absorbing/scattering particles may be used as a dispersion in an oil. The oil may be any of the silicone oils, the hydrocarbon oils, the ester oils, the natural animal and plant oils, the semi-synthetic oils, and the fluorochemical oils that are described hereinabove as the optional ingredients, namely, the oils (1), and are liquid. Specific examples of the oil dispersions of the UV-absorbing/scattering particles include SPD series (product name), in particular, SPD-T5, T5L, Z5, Z5L, T6, Z6, T7, and Z7L manufactured by Shin-Etsu Chemical Co., Ltd.

(8) Other additives

Other additives include oil-soluble gelling agents, preservatives, antibacterial agents, antiperspirants, fragrances, salts, antioxidants, acidity regulators, chelating agents, refreshing agents, anti-inflammatory agents, skin modifying ingredients (such as brightening or whitening agents, cell activators, anti-skin-roughening agents, blood flow enhancing agents, skin astringents, and antiseborrheic agents), vitamins, amino acids, nucleic acids, hormones, and clathrate compounds.

• Oil-soluble gelling agents

Exemplary oil-soluble gelling agents include metal soaps, such as aluminum stearate, magnesium stearate, and zinc myristate; amino acid derivatives, such as lauroyl glutamic acid (labeling name, INCI: Lauroyl Glutamic Acid) and $\alpha,\gamma$-di-n-butylamine; dextrin fatty acid esters, such as dextrin palmitate (labeling name, INCI: Dextrin Palmitate), dextrin isostearate (labeling name, INCI: Dextrin Isostearate), dextrin myristate (labeling name, INCI: Dextrin Myristate), inulin stearate (labeling name, INCI: Stearoyl Inulin), and dextrin palmitate/ethylhexanoate (labeling name, INCI: Dextrin Palmitate/Ethylhexanoate); sucrose fatty acid esters, such as sucrose palmitate and sucrose stearate; fructooligosaccharide fatty acid esters, such as fructooligosaccharide stearate and fructooligosaccharide 2-ethylhexanoate; benzylidene derivatives of sorbitol, such as monobenzylidene sorbitol and dibenzylidene sorbitol; organo-modified clay minerals of hectorite, such as Disteardimonium Hectorite (INCI) and Stearalkonium Hectorite (INCI); and Stearalkonium Bentonite (INCI).

• Preservatives and antibacterial agents

Examples of the preservatives and the antibacterial agents include alkyl p-hydroxybenzoates, benzoic acid, sodium benzoate, sorbic acid, potassium sorbate, phenoxyethanol, imidazolidinyl urea, salicylic acid, isopropyl methyl phenol, phenol, p-chloro-m-cresol, hexachlorophene, benzalkonium chloride, chlorohexidine chloride, trichlorocarbanilide, iodopropynyl butylcarbamate, polylysine, photosensitizers, silver, and plant extracts.

• Antiperspirants

Examples of the antiperspirants include aluminum halohydrates, such as aluminum chlorohydrate, aluminum halides, such as aluminum chloride, aluminum allantoinate, tannic acid, persimmon tannin, aluminum potassium sulfate, zinc oxide, zinc p-phenolsulfonate, calcined alum, aluminum zirconium tetrachlorohydrate, and aluminum zirconium trichlorohydrex glycine. In particular, preferred ingredients that exert a higher effect include aluminum halohydrates, aluminum halides, and complexes or mixtures thereof with zirconium oxyhalides and zirconium hydroxyhalides (for example, aluminum zirconium tetrachlorohydrate and aluminum zirconium trichlorohydrex glycine).

• Fragrances

The fragrances include natural fragrances and synthetic fragrances. Examples of the natural fragrances include plant fragrances separated from flowers, leaves, stems, and pericarps; and animal fragrances, such as musk and civet. Examples of the synthetic fragrances include hydrocarbons, such as monoterpene; alcohols, such as aliphatic alcohols and aromatic alcohols; aldehydes, such as terpene aldehydes and aromatic aldehydes; ketones, such as alicyclic ketones; esters, such as terpene esters; lactones; phenols; oxides; nitrogen-containing compounds; and acetals.

• Salts

Examples of the salts include inorganic salts, organic acid salts, amine salts, and amino acid salts. Exemplary inorganic salts include sodium salts, potassium salts, magnesium salts, calcium salts, aluminum salts, zirconium salts, and zinc salts of inorganic acids, such as hydrochloric acid, sulfuric acid, carbonic acid, and nitric acid. Exemplary organic acid salts include salts of organic acids, such as acetic acid, dehydroacetic acid, citric acid, malic acid, succinic acid, ascorbic acid, and stearic acid. Exemplary amine salts and amino acid salts include salts of amines, such as triethanol amine, and salts of amino acids, such as glutamic acid. Also useful are salts of hyaluronic acid, chondroitin sulfate, aluminum zirconium glycine complex, and acid-alkali neutral salts used in cosmetic formulations.

• Antioxidants

Examples of the antioxidants include, but are not particularly limited to, carotenoid, ascorbic acid and salts thereof, ascorbyl stearate, tocophenol, tocophenol acetate, tocopherol, p-t-butylphenol, butyl hydroxyanisole, dibutyl hydroxytoluene, phytic acid, ferulic acid, thiotaurine, hypotaurine, sulfites, erythorbic acid and salts thereof, chlorogenic acid, epicatechin, epigallocatechin, epigallocatechin gallate, apigenin, kaempferol, myricetin, and quercetin. The antioxidants may be used singly, or two or more may be used in combination.

• Acidity regulators

Examples of the acidity regulators include lactic acid, citric acid, glycolic acid, succinic acid, tartaric acid, dl-malic acid, potassium carbonate, sodium hydrogencarbonate, and ammonium hydrogencarbonate.

• Chelating agents

Examples of the chelating agents include alanine, sodium EDTA, sodium polyphosphate, sodium metaphosphate, and phosphoric acid.

• Refreshing agents

Examples of the refreshing agents include L-menthol, camphor, and menthyl lactate.

• Anti-inflammatory agents

Examples of the anti-inflammatory agents include allantoin, glycyrrhizinic acid and salts thereof, glycyrrhetinic acid, stearyl glycyrrhetinate, tranexamic acid, and azulene.

• Skin modifying ingredients

Examples of the skin modifying ingredients include brightening or whitening agents, such as placenta extract, arbutin, glutathione, and Saxifraga Sarmentosa Extract; wrinkle improving agents, such as retinol and niacinamide; cell activators, such as royal jelly, photosensitizers, cholesterol derivatives, and bovine blood extracts; anti-skin-roughening agents; blood flow enhancing agents, such as vanillylamide nonylate, benzyl nicotinate, $\beta$-butoxyethyl nicotinate, capsaicin, zingerone, cantharides tincture, ichthammol, caffeine, tannic acid, $\alpha$-borneol, tocopherol nicotinate, inositol hexanicotinate, cyclandelate, cinnarizine, tolazoline, acetylcholine, verapamil, cepharanthine, and $\gamma$-oryzanol; skin astringents, such as zinc oxide and tannic acid; and antiseborrheic agents, such as sulfur and thianthol.

• Vitamins

Examples of the vitamins include vitamin A family, such as vitamin A oil, retinol, retinol acetate, and retinol palmitate; vitamin B family, specifically, vitamin B2 family, such as riboflavin, riboflavin butyrate, and flavin adenine nucleotide, vitamin B6 family, such as pyridoxine hydrochloride, pyridoxine dioctanoate, and pyridoxine tripalmitate, vitamin B12 and derivatives thereof, and vitamin B15 and derivatives thereof; vitamin C family, such as L-ascorbic acid, L-ascorbic acid dipalmitic acid ester, L-ascorbic acid-2-sodium sulfate, and dipotassium L-ascorbic acid phosphoric acid diester; vitamin D family, such as ergocalciferol and cholecalciferol; vitamin E family, such as $\alpha$-tocopherol, $\beta$-tocopherol, $\gamma$-tocopherol, dl-$\alpha$-tocopherol acetate, dl-$\alpha$-tocopherol nicotinate, and dl-$\alpha$-tocopherol succinate; nicotinic acids, such as nicotinic acid, benzyl nicotinate, and nicotinic amide; vitamin H; vitamin P; pantothenic acids, such as calcium pantothenate, D-pantothenyl alcohol, pantothenyl ethyl ether, and acetylpantothenyl ethyl ether; and biotin.

• Amino acids

Examples of the amino acids include glycine, valine, leucine, isoleucine, serine, threonine, phenylalanine, arginine, lysine, aspartic acid, glutamic acid, cystine, cysteine, methionine, and tryptophan.

• Nucleic acids

Examples of the nucleic acids include deoxyribonucleic acid.

• Hormones

Examples of the hormones include estradiol and ethenyl estradiol.

• Clathrate compounds

Examples of the clathrate compounds include cyclodextrin.

EXAMPLES

Hereinafter, the present invention will be described in detail by presenting Production Examples, Comparative Production Examples, and Examples and Comparative Examples of dispersions and cosmetics. However, the present invention is not limited to the following Examples. Unless otherwise specified, "%" in the compositions below means "% by mass" and indicates the mass percentage of an ingredient relative to the mass of the total of each example taken as 100% by mass. The amount (content) indicates the amount of the product described.

[Production Example 1: Preliminary dispersion]

A preliminary dispersion was prepared according to the formulation described in Table 1.

(Production method)

A: Ingredients (1) were mixed.

B: Ingredient (2) was added to the mixture obtained in A and dispersed using a bead mill.

[Table 1]

| Ingredients (%) | | Production Example 1 |
|---|---|---|
| (1) | (D) Cyclopentasiloxane | 48 |
| | (C) KF-6105 (Note 1) | 7 |
| (2) | (B) Metal soap-treated microparticulate titanium oxide (15 nm) | 45 |
| Total | | 100 |
| (Note 1) Shin-Etsu Chemical Co., Ltd.: Lauryl polyglyceryl-3 polydimethylsiloxyethyl dimethicone | | |

[Examples 1 to 8 and Comparative Examples 1 to 4: Dispersions]

The dispersions obtained below were evaluated as follows.

<Viscosity stability>

The viscosity was measured immediately after the preparation of the dispersion and after 2 weeks at 25°C, and the viscosity change rate was calculated. The viscosity for this evaluation was measured with a Brookfield viscometer (TVB-10, manufactured by Toki Sangyo Co., Ltd.) with spindle No. 3, at 25°C and 30 rpm for a measurement time of 30 seconds. Dispersions that were measured to have a viscosity above the upper limit of measurement were marked with "-" and were deemed to have failed.
The viscosity change rate (%) in viscosity is calculated from:

Viscosity change rate (%) = (viscosity [mPa·s] after 2 weeks at 25°C)/(viscosity [mPa·s] immediately after preparation) × 100.

Dispersions that had a viscosity after 2 weeks at 25°C above the upper limit of measurement were marked with "-".

[Example 1]

A recovery flask was charged with 150 g of the preliminary dispersion of Production Example 1 and 72 g of (A) ethylhexyl palmitate (kinematic viscosity at 25°C: 15 mm$^2$/s) and was then attached to an evaporator. The mixture was gradually heated under reduced pressure using an oil bath to distill off the ingredient (D). The composition of the resultant dispersion was analyzed and was found to include (C) lauryl polyglyceryl-3 polydimethylsiloxyethyl dimethicone: 6.9% by mass, (B) metal soap-treated microparticulate titanium oxide (15 nm): 44.4% by mass, (D) cyclopentasiloxane: 1.4% by mass, and (A) ethylhexyl palmitate: 47.3% by mass. The dispersion obtained had an initial viscosity of 445 mPa·s and the viscosity change rate after 2 weeks at 25°C was 157%.

[Example 2]

A recovery flask was charged with 150 g of the preliminary dispersion of Production Example 1 and 70 g of (A) KF-56A (diphenylsiloxyphenyl trimethicone: kinematic viscosity at 25°C: 15 mm$^2$/s) manufactured by Shin-Etsu Chemical Co., Ltd. and was then attached to an evaporator. The mixture was gradually heated under reduced pressure using an oil bath to distill off the ingredient (D). The composition of the resultant dispersion was analyzed and was found to include (C) lauryl polyglyceryl-3 polydimethylsiloxyethyl dimethicone: 6.5% by mass, (B) metal soap-treated microparticulate titanium oxide (15 nm): 41.9% by mass, (D) cyclopentasiloxane: 8.2% by mass, and (A) KF-56A: 43.4% by mass. The dispersion obtained had an initial viscosity of 550 mPa·s and the viscosity change rate after 2 weeks at 25°C was 136%.

[Example 3]

A recovery flask was charged with the dispersion of Example 1 and was then attached to an evaporator. The dispersion was gradually heated under reduced pressure using an oil bath to further distill off the ingredient (D). The composition of the resultant dispersion was analyzed and was found to include (C) lauryl polyglyceryl-3 polydimethylsiloxyethyl dimethicone: 7% by mass, (B) metal soap-treated microparticulate titanium oxide (15 nm): 45% by mass, (A) ethylhexyl palmitate (kinematic viscosity at 25°C: 15 mm$^2$/s): 48% by mass, and (D) cyclopentasiloxane: 100 ppm. The dispersion obtained had an initial viscosity of 990 mPa·s and the viscosity change rate after 2 weeks at 25°C was 182%.

[Example 4]

A sample mill was charged with 10% by mass of (D) cyclopentasiloxane and 7% by mass of (C) lauryl polyglyceryl-3 polydimethylsiloxyethyl dimethicone. The mixture was stirred. Subsequently, 45% by mass of (B) metal soap-treated microparticulate titanium oxide (15 nm) was added and the mixture was further stirred to disperse the ingredient (B). To the dispersion, 38% by mass of (A) cocoalkyl (caprylate/caprate) (kinematic viscosity at 25°C: 5 mm$^2$/s) was admixed. The mixture was dispersed using a paint shaker. The dispersion obtained had an initial viscosity of 400 mPa·s and the viscosity change rate after 2 weeks at 25°C was 195%.

[Example 5]

A sample mill was charged with 7% by mass of (D) isododecane and 7% by mass of (C) lauryl polyglyceryl-3 polydimethylsiloxyethyl dimethicone. The mixture was stirred. Subsequently, 45% by mass of (B) metal soap-treated microparticulate titanium oxide (15 nm) was added and the mixture was further stirred to disperse the ingredient (B). To the dispersion, 40% by mass of (A) isononyl isononanoate (kinematic viscosity at 25°C: 7 mm$^2$/s) was admixed. The mixture was dispersed using a paint shaker. The dispersion obtained had an initial viscosity of 580 mPa·s and the viscosity change rate after 2 weeks at 25°C was 190%.

[Example 6]

A beaker was charged with 5% by mass of (D) C13-15 alkane and 7% by mass of (C) lauryl polyglyceryl-3 polydimethylsiloxyethyl dimethicone. The mixture was stirred. Subsequently, 43% by mass of (A) squalane (kinematic viscosity at 25°C: 30 mm$^2$/s) was added to give a mixed solution. To the mixed solution, 45% by mass of (B) metal soap-treated microparticulate titanium oxide (15 nm) was admixed. The mixture was dispersed using a paint shaker. The dispersion obtained had an initial viscosity of 378 mPa·s and the viscosity change rate after 2 weeks at 25°C was 165%.

[Example 7]

A beaker was charged with 8.2% by mass of (D) cyclopentasiloxane and 6.5% by mass of (C) lauryl polyglyceryl-3 polydimethylsiloxyethyl dimethicone. The mixture was stirred. Subsequently, 43.4% by mass of (A) ethylhexyl palmitate (kinematic viscosity at 25°C: 15 mm$^2$/s) was added to give a mixed solution. To the mixed solution, 41.9% by mass of (B) metal soap-treated microparticulate titanium oxide (15 nm) was admixed. The mixture was dispersed using a bead mill. The dispersion obtained had an initial viscosity of 449 mPa·s and the viscosity change rate after 2 weeks at 25°C was 212%.

[Example 8]

A beaker was charged with 1.4% by mass of (D) cyclopentasiloxane and 6.9% by mass of (C) lauryl polyglyceryl-3 polydimethylsiloxyethyl dimethicone. The mixture was stirred. Subsequently, 47.3% by mass of (A) diphenylsiloxyphenyl trimethicone (kinematic viscosity at 25°C: 15 mm$^2$/s) was added to give a mixed solution. To the mixed solution, 44.4% by mass of (B) metal soap-treated microparticulate titanium oxide (15 nm) was admixed. The mixture was dispersed using a bead mill. The dispersion obtained had an initial viscosity of 589 mPa·s and the viscosity change rate after 2 weeks at 25°C was 244%.

In the above Examples, the ingredient (C) is soluble in the ingredient (A).

[Comparative Examples 1 to 4: Dispersions]

Dispersions of Comparative Examples 1 to 4 were prepared according to the formulations described in Table 2.

(Production method)

A: Ingredients (1) were mixed.
B: Ingredient (2) was added to the mixture obtained in A and dispersed using a bead mill.

[Table 2]

| Ingredients (% by mass) | | | Comparative Example | | | |
|---|---|---|---|---|---|---|
| | | | 1 | 2 | 3 | 4 |
| (1) | (A) Ethylhexyl palmitate | | 48.0 | 48.0 | 48.0 | |
| | (D) Cyclopentasiloxane | | | | | 48.0 |
| | (C) KF-6105 (Note 1) | | 7.0 | | | |
| | KF-6100 (Note 2) | | | 7.0 | | |
| | (C) Polyhydroxystearic acid (Note 3) | | | | 7.0 | 7.0 |
| (2) | (B) Metal soap-treated microparticulate titanium oxide (15 nm) | | 45.0 | 45.0 | 45.0 | 45.0 |
| Total | | | 100.0 | 100.0 | 100.0 | 100.0 |
| Initial viscosity (mPa·s) | | | 1,826 | - | - | - |
| Viscosity change rate (%) | | | - | - | - | - |

| (Note 1) | Shin-Etsu Chemical Co., Ltd.: | Lauryl polyglyceryl-3 polydimethylsiloxyethyl dimethicone (soluble in ingredient (A)) |
|---|---|---|
| (Note 2) | Shin-Etsu Chemical Co., Ltd.: | Polyglyceryl-3 disiloxane dimethicone (insoluble in ingredient (A)) |
| (Note 3) | (Soluble in ingredient (A)) | |

The dispersions obtained in Examples 1 to 8 had a low initial viscosity. In particular, the dispersions of Examples 1 to 6 had a viscosity change rate after 2 weeks of less than 200% and were shown to have good dispersion stability. In contrast, the initial viscosity was high and dispersion properties were poor in the dispersions of Comparative Examples 1 and 3 that did not contain the ingredient (D), the dispersion of Comparative Example 4 that did not contain the ingredient (A), and the dispersion of Comparative Example 2 that did not contain the ingredient (D) and involved a dispersant insoluble in the ingredient (A).

[Examples 9 to 14 and Comparative Examples 5 to 8] (Cosmetics)

Oil-in-water (O/W) cosmetics were prepared according to the formulations described in Table 3. The oil-in-water (O/W) cosmetics obtained were evaluated as described later.

(Production method)

A: Ingredients (1) were mixed.
B: Ingredients (2) were mixed.
C: The mixture obtained in B was added to the mixture obtained in A and emulsified to give an oil-in-water (O/W) cosmetic.

[Properties of cosmetics]

Properties of the cosmetics obtained were evaluated by sandwiching the cosmetic between microscope slides. The cosmetics were judged to be "good" (acceptable) when properties were good and the bulk was continuous, and were judged to be "poor" (rejected) when the emulsification was poor and the bulk was not continuous.

[Over-time stability of cosmetics]

The cosmetic obtained was added into a 30 mL vial and was left to stand at 50°C. The over-time stability was judged as "good" (acceptable) when the cosmetic one month later still had good properties compared to the initial condition, as "fairly good" (acceptable) when the cosmetic one week later had good properties but the condition became poor after one month, and as "poor" (rejected) when the cosmetic one week later had poor properties. Those cosmetics that showed a poor condition in the initial stage were not tested for over-time stability.

[Table 3]

| Ingredients (%) | | | Example | | | | | |
|---|---|---|---|---|---|---|---|---|
| | | | 9 | 10 | 11 | 12 | 13 | 14 |
| (1) | Water | | 53 | 53 | 53 | 53 | 53 | 53 |
| | BG | | 5 | 5 | 5 | 5 | 5 | 5 |
| | Ethanol | | 10 | 10 | 10 | 10 | 10 | 10 |
| | PEG-20 sorbitan cocoate | | 1 | 1 | 1 | 1 | 1 | 1 |
| | Acrylic copolymer composition (Note 1) | | 2 | 2 | 2 | 2 | 2 | 2 |
| (2) | Cyclopentasiloxane | | 7.2 | 7.2 | 7.2 | 7.2 | 7.2 | 7.2 |
| | Ethylhexyl methoxycinnamate | | 6.8 | 6.8 | 6.8 | 6.8 | 6.8 | 6.8 |
| | Dispersion of Example 1 | | 15 | | | | | |
| | Dispersion of Example 2 | | | 15 | | | | |
| | Dispersion of Example 3 | | | | 15 | | | |
| | Dispersion of Example 4 | | | | | 15 | | |
| | Dispersion of Example 5 | | | | | | 15 | |
| | Dispersion of Example 6 | | | | | | | 15 |
| Total | | | 100.0 | 100.0 | 100.0 | 100.0 | 100.0 | 100.0 |
| Properties of cosmetic | | | Good | Good | Good | Good | Good | Good |
| Over-time stability of cosmetic | | | Good | Good | Good | Fairly good | Fairly good | Fairly good |

[Table 4]

| Ingredients (% by mass) | | Comparative Example | | | |
|---|---|---|---|---|---|
| | | 5 | 6 | 7 | 8 |
| (1) | Water | 53 | 53 | 53 | 53 |
| | BG | 5 | 5 | 5 | 5 |
| | Ethanol | 10 | 10 | 10 | 10 |
| | PEG-20 sorbitan cocoate | 1 | 1 | 1 | 1 |
| | Acrylic copolymer composition (Note 1) | 2 | 2 | 2 | 2 |
| (2) | Cyclopentasiloxane | 7.2 | 7.2 | 7.2 | 7.2 |
| | Ethylhexyl methoxycinnamate | 6.8 | 6.8 | 6.8 | 6.8 |
| | Dispersion of Comparative Example 1 | 15 | | | |
| | Dispersion of Comparative Example 2 | | 15 | | |
| | Dispersion of Comparative Example 3 | | | 15 | |
| | Dispersion of Comparative Example 4 | | | | 15 |
| Total | | 100.0 | 100.0 | 100.0 | 100.0 |
| Properties of cosmetic | | Poor | Poor | Poor | Poor |
| (Note 1) SEPPIC: SIMULGEL EG | | | | | |

As clear from the results in Tables 3 and 4, the dispersions of the present invention had good adsorption of the dispersant and successfully afforded cosmetics that had good properties and maintained dispersion properties and dispersion stability in spite of the extreme formulation involving an organic ultraviolet absorber.

[Production Examples 2 to 4: Preliminary dispersions]

Preliminary dispersions were prepared according to the formulations described in Table 5.

(Production method)

A: Ingredients (1) were mixed.
B: Ingredient (2) was added to the mixture obtained in A and dispersed using a bead mill.

[Table 5]

| Ingredients (% by mass) | | Production Example | | |
|---|---|---|---|---|
| | | 2 | 3 | 4 |
| (1) | (D) Dimethicone (1.5 cs) | 48 | | |
| | (D) Isododecane | | 48 | |
| | (D) Trisiloxane | | | 35 |
| | (C) KP-578 (Note 1) | 7 | | |
| | (C) KF-6115 (Note 2) | | 7 | |
| | (C) KF-6106 (Note 3) | | | 5 |
| (2) | (B) Metal soap-treated microparticulate titanium oxide (8 nm) | 45 | | |
| | (B) Hydrogen dimethicone-treated microparticulate titanium oxide (15 nm) | | 45 | |
| | (B) Hydrogen dimethicone-treated microparticulate zinc oxide (50 nm) | | | 60 |

(continued)

| Ingredients (% by mass) | Production Example | | |
|---|---|---|---|
| | 2 | 3 | 4 |
| Total | 100 | 100 | 100 |

(Note 1)  Shin-Etsu Chemical Co., Ltd.:  (Acrylates/ethylhexyl acrylate/dimethicone methacrylate) copolymer
(Note 2)  Shin-Etsu Chemical Co., Ltd.:  Lauryl polyglyceryl-3 polydimethylsiloxyethyl dimethicone
(Note 3)  Shin-Etsu Chemical Co., Ltd.:  Polyglyceryl-3 polydimethylsiloxyethyl dimethicone

[Production Examples 5 to 7: Preliminary dispersions]

Preliminary dispersions were prepared according to the formulations described in Table 6.

(Production method)

A: Ingredients (1) were mixed.
B: Ingredient (2) was added to the mixture obtained in A and dispersed using a high-pressure disperser.

[Table 6]

| Ingredients (% by mass) | | Production Example | | |
|---|---|---|---|---|
| | | 5 | 6 | 7 |
| (1) | (D) Methyl trimethicone | 48 | | |
| | (D) Disiloxane | | 48 | |
| | (D) Undecane | | | 35 |
| | (C) KP-578 (Note 1) | 7 | | |
| | (C) KF-6106 (Note 2) | | 7 | |
| | (C) KF-6115 (Note 3) | | | 5 |
| (2) | (B) Isostearic acid-treated microparticulate titanium oxide (10 nm) | 45 | | |
| | (B) Hydrogen dimethicone-treated microparticulate titanium oxide (80 nm) | | 45 | |
| | (B) Triethoxycaprylylsilane-treated microparticulate zinc oxide (35 nm) | | | 60 |
| Total | | 100 | 100 | 100 |

(Note 1)  Shin-Etsu Chemical Co., Ltd.:  (Acrylates/ethylhexyl acrylate/dimethicone methacrylate) copolymer
(Note 2)  Shin-Etsu Chemical Co., Ltd.:  Lauryl polyglyceryl-3 polydimethylsiloxyethyl dimethicone
(Note 3)  Shin-Etsu Chemical Co., Ltd.:  Polyglyceryl-3 polydimethylsiloxyethyl dimethicone

[Production Examples 8 to 10: Preliminary dispersions]

Preliminary dispersions were prepared according to the formulations described in Table 7.

(Production method)

A: Ingredients (1) were mixed.

B: Ingredients (2) were added to the mixture obtained in A and dispersed using a high-pressure disperser.

[Table 7]

| Ingredients (% by mass) | | | Production Example | | |
|---|---|---|---|---|---|
| | | | 8 | 9 | 10 |
| (1) | | (D) Cyclopentasiloxane | 48 | 48 | |
| | | (C) KF-6028 (Note 1) | 7 | | |
| | | (C) KF-6038 (Note 2) | | 7 | |
| | | (C) KF-6105 (Note 3) | | | 7 |
| (2) | | (B) Silicone-treated titanium oxide (Note 4) | 38 | 38 | 38 |
| | | (B) Silicone-treated iron oxide (Note 5) | 7 | 7 | 7 |
| Total | | | 100 | 100 | 100 |

(Note 1)    Shin-Etsu Chemical Co., Ltd.:    PEG-9 polydimethylsiloxyethyl dimethicone
(Note 2)    Shin-Etsu Chemical Co., Ltd.:    Lauryl PEG-9 polydimethylsiloxyethyl dimethicone
(Note 3)    Shin-Etsu Chemical Co., Ltd.:    Lauryl polyglyceryl-3 polydimethylsiloxyethyl dimethicone
(Note 4)    Shin-Etsu Chemical Co., Ltd.:    KTP-09W
(Note 5)    Shin-Etsu Chemical Co., Ltd.:    KTP-09R, Y, B

[Examples 15 to 23: Dispersions]

Dispersions were prepared in the same manner as in Example 1, except that the ingredient (A) and the preliminary dispersion were changed as described in the table below, and the amount of the ingredient (D) was adjusted to 5% by mass. The dispersions obtained had a viscosity change rate after 2 weeks at 25°C of less than 200%. In these Examples, the ingredient (C) is soluble in the ingredient (A).

[Table 8]

| Example | Ingredient (A) | Preliminary dispersion |
|---|---|---|
| 15 | Cocoalkyl (caprylate/caprate) | Preliminary dispersion of Production Example 2 |
| 16 | Glyceryl tri(caprylate/caprate) | Preliminary dispersion of Production Example 3 |
| 17 | Octyldodecyl myristate | Preliminary dispersion of Production Example 4 |
| 18 | Octyldodecyl myristate | Preliminary dispersion of Production Example 5 |
| 19 | Glyceryl tri(caprylate/caprate) | Preliminary dispersion of Production Example 6 |
| 20 | Cocoalkyl (caprylate/caprate) | Preliminary dispersion of Production Example 7 |
| 21 | Octyldodecyl myristate | Preliminary dispersion of Production Example 8 |
| 22 | Glyceryl tri(caprylate/caprate) | Preliminary dispersion of Production Example 9 |
| 23 | Cocoalkyl (caprylate/caprate) | Preliminary dispersion of Production Example 10 |

[Example 24] O/W sunscreen cream

| | Composition | % |
|---|---|---|
| 1. | Dispersion of Example 15 | 11 |
| 2. | Ethylhexyl methoxycinnamate | 5 |

(continued)

| | Composition | % |
|---|---|---|
| 3. | KF-56A (Note 1) | 3 |
| 4. | Cetanol | 0.5 |
| 5. | Polyoxyethylene sorbitan monooleate | 2.5 |
| 6. | Glyceryl stearate (SE) | 0.5 |
| 7. | KF-6011P (Note 2) | 1 |
| 8. | Dipropylene glycol | 6 |
| 9. | 1,3-Butylene glycol | 6 |
| 10. | Carboxyvinyl polymer | 0.3 |
| 11. | Acrylic polymer compound | 0.3 |
| 12. | Methylparaben | 0.2 |
| 13. | Phenoxyethanol | 0.3 |
| 14. | Disodium EDTA | q.s. |
| 15. | Water | Balance |
| 16. | 10% Aqueous sodium hydroxide solution | q.s. |
| | Total | 100.0 |

(Note 1)     Shin-Etsu Chemical Co., Ltd.:     Diphenylsiloxyphenyl trimethicone
(Note 2)     Shin-Etsu Chemical Co., Ltd.:     PEG-11 methyl ether dimethicone

(Production method)

A: Ingredients 1 to 4 were mixed uniformly at 90°C.
B: Ingredients 5 to 15 were mixed uniformly at 85°C.
C: The mixture obtained in A was added to the mixture obtained in B and emulsified, and ingredient 16 was added. The mixture was mixed uniformly to give an O/W sunscreen.

The O/W sunscreen obtained had high transparency, was less sticky, and had a good feeling on use.

[Example 25] Oil-in-water sunscreen

| | Composition | % |
|---|---|---|
| 1. | KSG-15 (Note 1) | 8.0 |
| 2. | KSG-16 (Note 2) | 24.0 |
| 3. | Cyclopentasiloxane | 8.0 |
| 4. | Ethylhexyl methoxycinnamate | 2.0 |
| 5. | BG | 3.0 |
| 6. | KF-6100 (Note 3) | 0.6 |
| 7. | KF-6104 (Note 4) | 0.3 |
| 8. | Aqueous solution of (ammonium acryloyldimethyltaurate/VP) copolymer (Note 5) | 13.0 |
| 9. | Aqueous solution of (acrylamide/sodium acryloyldimethyltaurate) copolymer/isohexadecane/polysorbate 80 (Note 6) | 0.6 |
| 10. | 1% Aqueous sodium chloride solution | 8.0 |
| 11. | Water | 12.5 |
| 12. | Dispersion of Example 1 | 10.0 |
| 13. | Dispersion of Example 17 | 10.0 |

(continued)

| Composition | % |
|---|---|
| Total | 100.0 |

| (Note 1) | Shin-Etsu Chemical Co., Ltd.: | Mixture of 90-96% cyclopentasiloxane + 4-10% (dimethicone/vinyl dimethicone) crosspolymer |
|---|---|---|
| (Note 2) | Shin-Etsu Chemical Co., Ltd.: | Mixture of 70-80% dimethicone + 20-30% (dimethicone/vinyl dimethicone) crosspolymer |
| (Note 3) | Shin-Etsu Chemical Co., Ltd.: | Polyglyceryl-3 disiloxane dimethicone |
| (Note 4) | Shin-Etsu Chemical Co., Ltd.: | Polyglyceryl-3 polydimethylsiloxyethyl dimethicone |
| (Note 5) | Clariant: | Aristoflex AVC |
| (Note 6) | SEPPIC: | SIMULGEL 600 |
| (Note 7) | Shin-Etsu Chemical Co., Ltd.: | Treated with KF-9901 (hydrogen dimethicone) |
| (Note 8) | Shin-Etsu Chemical Co., Ltd.: | Treated with AES-3083 (triethoxycaprylylsilane) |

(Production method)

A: Ingredients 1 to 4 were mixed uniformly.

B: Ingredients 5 to 11 were mixed uniformly.

C: The mixture obtained in A was added to the mixture obtained in B and emulsified, and furthermore ingredients 12 and 13 were added and dispersed uniformly.

The oil-in-water sunscreen obtained as described above was highly stable and highly transparent, gave a non-sticky and comfortable feeling on use, and had excellent water resistance.

[Example 26] Aqueous gel

| | Composition | % |
|---|---|---|
| 1. | Dispersion of Example 18 | 5.0 |
| 2. | Ethanol | 3.5 |
| 3. | BG | 4.0 |
| 4. | Glycerin | 2.0 |
| 5. | (Ammonium acryloyldimethyltaurate/VP) copolymer | 0.2 |
| 6. | Xanthan gum | 0.2 |
| 7. | Arginine | 0.5 |
| 8. | Phenoxyethanol | 0.3 |
| 9. | Water | Balance |
| | Total | 100.0 |

(Production method)

A: Ingredients 1 and 2 were mixed uniformly.

B: Ingredients 3 to 9 were mixed uniformly.

C: The mixture obtained in the above step A was added to the mixture obtained in the above step B. The resultant mixture was mixed uniformly.

D: The mixture obtained in the above step C was degassed and then added into a container. An aqueous gel was thus obtained.

The aqueous gel obtained as described above was very dewy when applied, had high stability and high transparency, gave a non-sticky and comfortable feeling on use, and had excellent water resistance.

[Example 27] Oil-in-water base cream

| | Composition | % |
|---|---|---|
| 1. | Water | B Balance |
| 2. | Glycerin | 3.0 |
| 3. | Microcrystalline wax | 3.0 |
| 4. | Xanthan gum | 0.2 |
| 5. | Pentylene glycol | 2.0 |
| 6. | BG | 5.0 |
| 7. | Dispersion of Example 2 | 10.0 |
| 8. | Sucrose cocoate | 0.2 |
| 9. | Sorbitan stearate | 3.0 |
| 10. | PEG-60 glyceryl isostearate | 0.5 |
| 11. | Behenyl alcohol | 0.5 |
| 12. | Ethylhexyl palmitate | 3.0 |
| 13. | KSP-101 (Note 1) | 3.0 |
| 14. | Dispersion of Example 23 | 10.0 |
| 15. | Polysorbate 60 | 0.3 |
| 16. | (Hydroxyethyl acrylate/sodium acrvlovldimethvltaurate) copolymer (Note 2) | 0.6 |
| | Total | 100.0 |

| | | |
|---|---|---|
| (Note 1) | Shin-Etsu Chemical Co., Ltd.: | (vinyl dimethicone/methicone silsesquioxane) crosspolymer |
| (Note 2) | SEPPIC: | SIMULGEL EG |

(Production method)

A: Ingredients 1 to 6 were mixed uniformly, and ingredient 7 was further added. The mixture was mixed uniformly.

B: Ingredients 8 to 12 were heated to give a solution, and ingredient 13 was further added. The mixture was mixed uniformly.

C: Ingredient 14 was added to the mixture obtained in the above step B. The resultant mixture was mixed uniformly.

D: The mixture obtained in the above step C was heated and was added to the mixture from the above step A that had been heated. The resultant mixture was emulsified uniformly.

E: The emulsion obtained in the above step D was cooled to room temperature, and subsequently ingredients 24 and 25 were added and mixed uniformly.

F: The mixture obtained in the above step E was degassed and was added into a container. An oil-in-water base cream was thus obtained.

The oil-in-water base cream obtained as described above was highly stable and highly transparent, gave a non-sticky and comfortable feeling on use, and had excellent water resistance.

[Example 28] Oil-in-water sunscreen milky lotion

| | Composition | % |
|---|---|---|
| 1. | Dispersion of Example 3 | 15.2 |
| 2. | Ethanol | 9.5 |
| 3. | BG | 5.0 |
| 4. | Methylparaben | 0.1 |
| 5. | Sodium acrylate/sodium acryloyldimethyltaurate copolymer composition (Note 1) | 2.5 |
| 6. | Water | Balance |
| 7. | KF-7312J (Note 2) | 1.0 |
| 8. | KF-56A (Note 3) | 3.0 |
| 9. | KSG-016F (Note 4) | 1.0 |
| 10. | Cetanol | 2.0 |
| 11. | Ethylhexyl methoxycinnamate | 5.0 |
| 12. | Diethylamino hydroxybenzoyl hexyl benzoate | 1.0 |
| 13. | Polyoxyethylene (60) hydrogenated castor oil | 1.0 |
| 14. | KF-6011 (Note 5) | 0.5 |
| 15. | Tocopherol | 0.05 |
| | Total | 100.0 |

| (Note 1) | SEPPIC: | SIMULGEL EG |
|---|---|---|
| (Note 2) | Shin-Etsu Chemical Co., Ltd.: | 50% Solution of trimethylsiloxysilicic acid in cyclopentasiloxane |
| (Note 3) | Shin-Etsu Chemical Co., Ltd.: | Diphenylsiloxyphenyl trimethicone |
| (Note 4) | Shin-Etsu Chemical Co., Ltd.: | Mixture of 70-80% dimethicone + 20-30% (dimethicone/vinyl dimethicone) crosspolymer |
| (Note 5) | Shin-Etsu Chemical Co., Ltd.: | PEG-11 methyl ether dimethicone |

(Production method)

A: Ingredients 2 to 6 were heated to 85°C, and ingredient 1 was added. The resultant mixture was mixed uniformly.

B: Ingredients 7 to 15 were heated to 85°C and were mixed uniformly.

C: The mixture obtained in B was added to the mixture obtained in A and emulsified at 85°C. While performing stirring, the emulsion was cooled slowly. An oil-in-water sunscreen milky lotion was thus obtained.

The oil-in-water sunscreen milky lotion obtained as described above was highly stable and highly transparent, gave a non-sticky and comfortable feeling on use, and had excellent water resistance.

[Example 29] Oil-in-water base

| | Composition | % |
|---|---|---|
| 1. | KP-545 (Note 1) | 3 |
| 2. | KSG-19 (Note 2) | 5 |

(continued)

| Composition | | %  |
|---|---|---|
| 3. | KF-56A (Note 3) | 5 |
| 4. | Cyclopentasiloxane | 10 |
| 5. | Dispersion of Example 3 | 30.6 |
| 6. | BG | 10 |
| 7. | Betaine | 1 |
| 8. | KF-6043 (Note 4) | 1.5 |
| 9. | Sodium acrylate/sodium acryloyldimethyltaurate copolymer composition (Note 5) | 1 |
| 10. | (Acrylates/alkyl (C10-30) acrylate) crosspolymer (2% aqueous solution) | 20 |
| 11. | Arginine (10% aqueous solution) | q.s. |
| 12. | Bisabolol | 0.1 |
| 13. | Ethylhexylglycerin | 0.1 |
| 14. | EDTA-2Na (10% aqueous solution) | 0.1 |
| 15. | Water | Balance |
| | Total | 100.0 |

| (Note 1) | Shin-Etsu Chemical Co., Ltd.: | 30% Solution of (acrylates/dimethicone) copolymer in cyclopentasiloxane |
|---|---|---|
| (Note 2) | Shin-Etsu Chemical Co., Ltd.: | Mixture of 80-90% dimethicone + 10-20% (dimethicone/vinyl dimethicone) crosspolymer |
| (Note 3) | Shin-Etsu Chemical Co., Ltd.: | Diphenylsiloxyphenyl trimethicone |
| (Note 4) | Shin-Etsu Chemical Co., Ltd.: | PEG-10 dimethicone |
| (Note 5) | SEPPIC: | SIMULGEL EG |

(Production method)

A: Ingredients 1 to 4 were mixed uniformly.

B: Ingredients 6 to 15 and 5 were mixed uniformly.

C: The mixture obtained in A was added to the mixture obtained in B and emulsified to give an O/W base.

The oil-in-water base obtained as described above was highly stable and highly transparent, gave a non-sticky and comfortable feeling on use, and had excellent water resistance.

[Example 30] Oil-in-water liquid foundation

| Composition | | %  |
|---|---|---|
| 1. | Stearic acid | 1.0 |
| 2. | Behenyl alcohol | 0.4 |
| 3. | Glyceryl stearate | 0.3 |
| 4. | Mineral oil | 10.0 |
| 5. | Glyceryl trioctanoate | 5.0 |
| 6. | KP-561P (Note 1) | 3.0 |
| 7. | Sorbitan sesquioleate | 0.5 |
| 8. | Sorbitan monooleate | 1.0 |
| 9. | Acrylates copolymer | 2.2 |

(continued)

| | Composition | % |
|---|---|---|
| 10. | Triethanolamine | 1.0 |
| 11. | KF-6106 (Note 2) | 1.0 |
| 12. | KP-545 (Note 3) | 0.5 |
| 13. | Isotridecyl isononanoate | 4.0 |
| 14. | Silicone-treated titanium oxide (Note 4) | 8.5 |
| 15. | Silicone-treated red iron oxide (Note 4) | 0.4 |
| 16. | Silicone-treated yellow iron oxide (Note 4) | 1.0 |
| 17. | Silicone-treated black iron oxide (Note 4) | 0.1 |
| 18. | Dispersion of Example 16 | 12.0 |
| 22. | BG | 7.0 |
| 23. | Preservative | q.s. |
| 24. | Fragrance | q.s. |
| 25. | Water | Balance |
| | Total | 100.0 |

(Note 1)　Shin-Etsu Chemical Co., Ltd.:　(Acrylates/stearyl acrylate/dimethicone methacrylate) copolymer
(Note 2)　Shin-Etsu Chemical Co., Ltd.:　Polyglyceryl-3 polydimethylsiloxyethyl dimethicone
(Note 3)　Shin-Etsu Chemical Co., Ltd.:　30% Solution of (acrylates/dimethicone) copolymer in cyclopentasiloxane
(Note 4)　Shin-Etsu Chemical Co., Ltd.:　Treated with triethoxycaprylylsilane

(Production method)

A: Ingredients 11 to 13 were mixed, and ingredients 14 to 17 were added. The resultant mixture was rolled.

B: Ingredients 1 to 8 were mixed and heated to give a uniform solution.

C: Ingredients 9, 10, 22, 23, and 25 were mixed and heated.

D: While performing stirring, the mixture obtained in B was added to the mixture obtained in C and emulsified. The mixture obtained in A was added to the emulsion, and furthermore ingredients 18 and 24 were added. An oil-in-water liquid foundation was thus obtained.

The oil-in-water liquid foundation obtained as described above was highly stable and highly transparent, gave a non-sticky and comfortable feeling on use, and had excellent water resistance.

[Example 31] Water-in-oil sunscreen milky lotion

| | Composition | % |
|---|---|---|
| 1. | KSG-210 (Note 1) | 3.0 |
| 2. | KSG-15 (Note 2) | 2.0 |
| 3. | KF-6028 (Note 3) | 1.0 |
| 4. | Dimethicone 6 CS | 5.0 |
| 5. | Cyclopentasiloxane | 5.0 |
| 6. | Isotridecyl isononanoate | 4.0 |
| 7. | Dispersion of Example 3 | 25.0 |
| 8. | Dispersion of Example 20 | 35.0 |
| 9. | Sodium citrate | 0.2 |

(continued)

| | Composition | % |
|---|---|---|
| 10. | Sodium chloride | 0.5 |
| 11. | Preservative | q.s. |
| 12. | Fragrance | q.s. |
| 13. | Water | Balance |
| | Total | 100.0 |

(Note 1)  Shin-Etsu Chemical Co., Ltd.:  Mixture of 70-80% dimethicone + 20-30% (dimethicone/(PEG-10/15)) crosspolymer

(Note 2)  Shin-Etsu Chemical Co., Ltd.:  Mixture of 90-96% cyclopentasiloxane + 4-10% (dimethicone/vinyl dimethicone) crosspolymer

(Note 3)  Shin-Etsu Chemical Co., Ltd.:  PEG-9 polydimethylsiloxyethyl dimethicone

(Production method)

A: Ingredients 1 to 6 were mixed uniformly.

B: Ingredients 9 to 13 were mixed uniformly.

C: While performing stirring, the mixture obtained in A was added to the mixture obtained in B and emulsified, and ingredients 7 and 8 were added. A water-in-oil sunscreen milky lotion was thus obtained.

The sunscreen milky lotion obtained as described above was highly stable and highly transparent, gave a non-sticky and comfortable feeling on use, and had excellent water resistance.

[Example 32] Sunscreen milky lotion (shaking)

| | Composition | % |
|---|---|---|
| 1. | Dimethylpolysiloxane (1.5 cs) | 22.5 |
| 2. | Dimethylpolysiloxane (6 cs) | 2 |
| 3. | Cyclopentasiloxane | 7 |
| 4. | KSG-18A (Note 1) | 3 |
| 5. | KF-6038 (Note 2) | 2 |
| 6. | Ethylhexyl methoxycinnamate | 7.5 |
| 7. | Diethylamino hydroxybenzoyl hexyl benzoate | 1 |
| 8. | Octocrylene | 2.5 |
| 9. | Disteardimonium hectorite | 1 |
| 10. | KP-545 (Note 3) | 2 |
| 11. | KSP-105 (Note 4) | 0.5 |
| 12. | Dispersion of Example 6 | 5 |
| 13. | Dispersion of Example 17 | 10 |
| 14. | BG | 3 |
| 15. | Sodium citrate | 0.2 |
| 16. | Sodium chloride | 0.5 |
| 17. | Ethanol | 5 |
| 18. | Water | 25.3 |
| | Total | 100.0 |

| (Note 1) | Shin-Etsu Chemical Co., Ltd.: | Mixture of 80-90% diphenylsiloxyphenyl trimethicone + 10-20% (dimethicone/phenyl vinyl dimethicone) crosspolymer |
| --- | --- | --- |
| (Note 2) | Shin-Etsu Chemical Co., Ltd.: | Lauryl PEG-9 polydimethylsiloxyethyl dimethicone |
| (Note 3) | Shin-Etsu Chemical Co., Ltd.: | Solution of 70% cyclopentasiloxane + 30% (acrylates/dimethicone) copolymer |
| (Note 4) | Shin-Etsu Chemical Co., Ltd.: | (Vinyl dimethicone/methicone silsesquioxane) crosspolymer |
| (Note 5) | Shin-Etsu Chemical Co., Ltd.: | 40% Dispersion of microparticulate titanium oxide in cyclopentasiloxane |
| (Note 6) | Shin-Etsu Chemical Co., Ltd.: | 60% Dispersion of microparticulate zinc oxide in cyclopentasiloxane |

(Production method)

A: Ingredients 1 to 10 were mixed uniformly.
B: Ingredient 11 was added to the mixture obtained in A and dispersed uniformly.
C: Ingredients 14 to 17 were added to ingredient 18 and dissolved.
D: The mixture obtained in C was gradually added to the mixture obtained in B and emulsified, and subsequently ingredients 12 and 13 were added. A sunscreen milky lotion was thus obtained.

The sunscreen milky lotion obtained as described above spread lightly, left the skin dry without stickiness, and was free from changes due to temperature or aging, thus being shown to have excellent usability and stability.

[Example 33]

A dispersion was prepared in the same manner as in Example 1, except that the ingredient (A) was changed to KF-4418 (caprylyl methicone) manufactured by Shin-Etsu Chemical Co., Ltd., the preliminary dispersion of Production Example 1 was replaced by the preliminary dispersion of Production Example 2, and the amount of the ingredient (D) was adjusted to 1,000 ppm. The dispersion obtained had a viscosity change rate after 2 weeks at 25°C of less than 200%.

[Example 34] Sun care stick

| | | Composition | % |
| --- | --- | --- | --- |
| 1. | | Dispersion of Example 33 | 6 |
| 2. | | KF-56A (Note 1) | Balance |
| 3. | | KMP-592 (Note 2) | 0.5 |
| 4. | | Diisopropyl sebacate | 10 |
| 5. | | Ethylhexyl methoxycinnamate | 7.5 |
| 6. | | Octocrylene | 2.5 |
| 7. | | Diethylamino hydroxybenzoyl hexyl benzoate | 5 |
| 8. | | Ethylhexyl salicylate | 5 |
| 9. | | Bis-ethylhexyloxyphenol methoxyphenyl triazine | 3 |
| 10. | | Polysilicone-15 | 2 |
| 11. | | Homosalate | 10 |
| 12. | | Dibutyl lauroyl glutamide | 3.5 |
| 13. | | Octyldodecanol | 10 |
| 14. | | Mineral oil | 2 |
| | | Total | 100.0 |

| (Note 1) | Shin-Etsu Chemical Co., Ltd.: | Diphenylsiloxyphenyl dimethicone |
|---|---|---|
| (Note 2) | Shin-Etsu Chemical Co., Ltd.: | (Methyl/phenyl) polysilsesquioxane |

(Production method)

A: Ingredients 1 to 14 were mixed uniformly at 110°C. The mixture was added into a stick container. A sun care stick was thus obtained.

The sun care stick obtained spread lightly, left the skin dry without stickiness, and was free from changes due to temperature or aging, thus being shown to have excellent usability and stability.

[Example 35] W/O stick base

| | Composition | % |
|---|---|---|
| 1. | KSG-830 (Note 1) | 5 |
| 2. | KSG-45 (Note 2) | 5 |
| 3. | KF-6105 (Note 2) | 0.5 |
| 4. | Meadowfoam oil | 2 |
| 5. | KF-56A (Note 3) | 6 |
| 6. | Ceresin | 9 0.2 |
| 7. | BG | 7 |
| 8. | PCA-Na | 2 |
| 9. | Diglycerin | 3 |
| 10. | Sodium citrate | 0.2 |
| 11. | Magnesium sulfate | 1 |
| 12. | Water | Balance |
| 13. | Dispersion of Example 17 | 5 |
| 14. | Dispersion of Example 19 | 5 |
| 15. | Dispersion of Example 23 | 5 |
| 16. | KSP-300 | 0.5 |
| | Total | 100.0 |

| (Note 1) | Shin-Etsu Chemical Co., Ltd.: | Mixture of 20% (lauryl dimethicone/polyglycerin-3) crosspolymer and 80% triethylhexanoin |
|---|---|---|
| (Note 2) | Shin-Etsu Chemical Co., Ltd.: | Mixture of 35% (vinyl dimethicone/lauryl dimethicone) crosspolymer and 65% cocoalkyl (caprylate/caprate) |
| (Note 3) | Shin-Etsu Chemical Co., Ltd.: | Lauryl PEG-9 polydimethylsiloxyethyl dimethicone |
| (Note 4) | Shin-Etsu Chemical Co., Ltd.: | Diphenylsiloxyphenyl trimethicone |

(Production method)

A: Ingredients 1 to 6 were mixed uniformly at 90°C.

B: Ingredients 7 to 12 were mixed uniformly at 85°C.

C: The mixture obtained in B was added to the mixture obtained in A and emulsified, and ingredients 13 to 16 were added. The resultant mixture was mixed. The mixture was added into a stick container. A W/O stick base was thus obtained.

The W/O stick base obtained spread lightly, left the skin dry without stickiness, and was free from changes due to temperature or aging, thus being shown to have excellent usability and stability.

**Claims**

1. A dispersion comprising ingredients (A), (B), (C), and (D) below:

   (A) a nonvolatile oil having a kinematic viscosity at 25°C of 1 to 100 mm$^2$/s: 15 to 65% by mass,
   (B) a powder: 30 to 70% by mass,
   (C) a dispersant soluble in the ingredient (A): 1 to 15% by mass, and
   (D) a volatile ingredient selected from lower alcohols and volatile oils having a boiling point of 250°C or below: 100 ppm to 10% by mass.

2. The dispersion according to claim 1, wherein the ingredient (B) is dispersed with the ingredients (C) and (D), and the ingredient (B) thus dispersed is further dispersed in the ingredient (A).

3. The dispersion according to claim 1, which has a viscosity change rate after 2 weeks at 25°C of less than 250% relative to a viscosity immediately after preparation.

4. The dispersion according to claim 1, wherein the ingredient (A) is one or more selected from ester oils, phenyl silicones, and alkyl silicones.

5. The dispersion according to claim 1, wherein the ingredient (B) has an average primary particle size of 8 to 200 nm.

6. The dispersion according to claim 1, wherein the ingredient (B) is hydrophobized microparticulate titanium oxide.

7. The dispersion according to claim 1, wherein the ingredient (C) is a silicone surfactant.

8. The dispersion according to claim 1, wherein the ingredient (D) comprises one or more selected from isododecane, undecane, tridecane, cyclopentasiloxane, disiloxane, trisiloxane, dimethicone, and methyltrimethicone.

9. A method for producing the dispersion described in any one of claims 1 to 8, the method comprising:

   a step of dispersing the ingredient (B) with the ingredients (C) and (D) to prepare a preliminary dispersion; and
   a step of mixing the preliminary dispersion with the ingredient (A).

10. A cosmetic comprising the dispersion described in any one of claims 1 to 8.

11. The cosmetic according to claim 10, wherein the cosmetic is an oil-in-water cosmetic.

12. The cosmetic according to claim 10, further comprising (E) an organic ultraviolet absorber.

## INTERNATIONAL SEARCH REPORT

| International application No. |
| --- |
| **PCT/JP2024/025657** |

**A. CLASSIFICATION OF SUBJECT MATTER**

*A61K 8/04*(2006.01)i; *A61K 8/19*(2006.01)i; *A61K 8/31*(2006.01)i; *A61K 8/34*(2006.01)i; *A61K 8/37*(2006.01)i; *A61K 8/58*(2006.01)i; *A61K 8/891*(2006.01)i; *A61K 8/894*(2006.01)i; *A61Q 17/04*(2006.01)i; *A61Q 19/00*(2006.01)i
FI:    A61K8/04; A61K8/37; A61K8/891; A61K8/19; A61K8/894; A61K8/31; A61K8/58; A61Q19/00; A61Q17/04; A61K8/34

According to International Patent Classification (IPC) or to both national classification and IPC

**B. FIELDS SEARCHED**

Minimum documentation searched (classification system followed by classification symbols)

A61K8/04; A61K8/19; A61K8/31; A61K8/34; A61K8/37; A61K8/58; A61K8/891; A61K8/894; A61Q17/04; A61Q19/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | WO 2017/199732 A1 (SHIN-ETSU CHEMICAL CO., LTD.) 23 November 2017 (2017-11-23) <br> entire text | 1-12 |
| A | WO 2019/176555 A1 (SHIN-ETSU CHEMICAL CO., LTD.) 19 September 2019 (2019-09-19) <br> entire text | 1-12 |
| A | WO 2020/017346 A1 (SHIN-ETSU CHEMICAL CO., LTD.) 23 January 2020 (2020-01-23) <br> entire text | 1-12 |
| A | WO 2021/241445 A1 (DNP FINE CHEMICALS CO., LTD.) 02 December 2021 (2021-12-02) <br> entire text | 1-12 |
| P, A | WO 2024/004579 A1 (SHIN-ETSU CHEMICAL CO., LTD.) 04 January 2024 (2024-01-04) <br> entire text | 1-12 |

☐ Further documents are listed in the continuation of Box C.    ☑ See patent family annex.

| | |
| --- | --- |
| \* Special categories of cited documents: <br> "A" document defining the general state of the art which is not considered to be of particular relevance <br> "D" document cited by the applicant in the international application <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
| --- | --- |
| **10 September 2024** | **24 September 2024** |

| Name and mailing address of the ISA/JP | Authorized officer |
| --- | --- |
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (July 2022)

**INTERNATIONAL SEARCH REPORT**
Information on patent family members

International application No.

**PCT/JP2024/025657**

| Patent document cited in search report | | | Publication date (day/month/year) | Patent family member(s) | | | Publication date (day/month/year) |
|---|---|---|---|---|---|---|---|
| WO | 2017/199732 | A1 | 23 November 2017 | JP | 2020-40994 | A | |
| WO | 2019/176555 | A1 | 19 September 2019 | US | 2021/0022984 | A1 | |
| | | | | whole document | | | |
| | | | | EP | 3766478 | A1 | |
| | | | | CN | 111867558 | A | |
| | | | | KR | 10-2020-0132928 | A | |
| WO | 2020/017346 | A1 | 23 January 2020 | (Family: none) | | | |
| WO | 2021/241445 | A1 | 02 December 2021 | (Family: none) | | | |
| WO | 2024/004579 | A1 | 04 January 2024 | (Family: none) | | | |

Form PCT/ISA/210 (patent family annex) (July 2022)

**EP 4 751 701 A1**

**REFERENCES CITED IN THE DESCRIPTION**

**Patent documents cited in the description**

- WO 2016178380 A
- WO 2017199732 A
- JP 3912961 B